# EUROPEAN PATENT APPLICATION

(11) **EP 4 194 009 A1**
(43) Date of publication of application: **14.06.2023**
(21) Application number: 22216911.2
(22) Date of filing: 13.02.2008
(51) Int. Cl.: A61K 41/00, C02F 1/00

(54) **TINNITUS TREATMENT DEVICE**

(30) Priority: 13.02.2007 SE 0700372
(62) Divisional of application: 10164290.8
(71) Applicant: Antinitus AB, 114 39 Stockholm (SE)
(72) Inventor: Johansson, Benny, 213 62 Malmö (SE)
(74) Representative: AWA Sweden AB

(57) **Abstract**

A tinnitus treatment device is disclosed, wherein it comprises a topographic geometrical matrix and a support, wherein the topographic geometrical matrix is applied on the support and has the form of an open circle concentrically enclosing a smaller inner circle (an SSc matrix), wherein the relationship between the circle diameter follows Fibonacchi's sequence of numbers, or has the form of the flower of life.

## Description

### Technical Field of the invention

The present invention relates to synchronized water, a method for production of the synchronized water, and different uses of said synchronized water.

### Background Art

Water is the third most common substance on earth and is the only naturally occurring liquid (1), and constitutes an essential component in all biological life. Water is unique and has a nuanced spectrum of anomalous properties (1-5). It can be ascertained that the biological life is dependent on the atypical properties of water (6). With a view to explaining the peculiar structure of water, as well as its physical and chemical properties in both microscopical and macroscopical view, a combination of different explanation models is required (1,2,7). A well disclosed conclusive model requires knowledge of the interaction between adjacent water molecules, the relationship between pressure and density and between temperature and density, and also explains the effect on dissolved substances in water (2). Essentially, the water could be disclosed starting with a wave cluster model defining the pleiotrophic nature of water on a pico-second time scale and consisting of microscopical permanent interactive water clusters in a permanent interaction and reorganisation of the localisation and migration of single water molecules, as well as instantaneous dissolution and re-formation of single hydrogen bonds (2,8).

Water clusters have a definable volume and size, which represents a balance between co-operative bonds holding the molecules together and collisions breaking them apart by external pressure on the microscopical level and internal tension from a microscopical view (9,10). The formation and breakage of hydrogen bonds between individual water molecules is co-operative, wherein the molecules act synergistically like a coherent field through the whole cluster network and are moved through the water mass as repeatable pulsing waves of polymerisation and depolymerisation reactions (11). The dynamic properties of the cluster could be compared with a biological living system, in which single (water) molecules permanently are moved and exchanged, while the geometrical conformation, structure and form of the cluster/system remain dynamically intact. Water clusters containing up to several hundreds of water molecules have been identified (9,10).

The water molecule is neutral as to charge and shows at the same time a powerful dipole moment due to the electron polarisation of the water molecule (12). In such a way the molecular charge symmetry is generated, resulting in attraction between single water molecules and charged ions dissolved in the water, which stimulates the formation of molecule swarms or molecular clusters. The powerful dipole moment allows for permanent reorientation, mobility and migration of the water molecules and is dependent on the emergency and synchronizity (13) in the common field-like electron configuration of the water system, which manifests the nuanced and anomalous water in relation to other liquids (2).

The basis for the molecular co-operativity within said water clusters is a high flexibility in defined micro-domains to convert between low and high density domains within the cluster (1,2,14). The micro-domains of the cluster are capable of a flexible partial exchange between low density (LDV) and high density (HDV) water (14,15,16). Due to the phase shift between LDV and HDV, the network of the cluster has a variable dynamic formability to create capacitive pores and cavities housing dissolved substances in the water. Water clusters having LDV and HDV properties of different sizes have been identified in both water solution and air (17,18). The driving force in the cluster-domain interaction between LDV and HDV clusters comprises macrol-microscopical (pressure-tension) hydration/dehydration control, which is operative in the interface between water and the dissolved substance. Said control is supported by pulsing osmotic energy liberated during the phase conversion between spatially expanding and compressed or collapsed clusters, respectively (19). The lateral tension in the water layer in the interface to hydrophilic minerals and biological substances is extremely high, more precisely up to 1000 atmospheres within a radius of 3 nm from the surface of the substance (19). The cluster model predicates for extended aggregating clusters, laterally expanding to macroscopical proportions (20).

Low-density water has a high structural stability with the hydrogen atom in a straight line between two oxygen atoms, which keep the molecules apart (7). High-density water is a molecularly compressed form, in which hydrogen bonds "are pressed together", but not broken, and allows molecular association increasing the cluster water density (7). Low-density water is characterized by more stable hydrogen bonds and lower (negative) entropy, i.e. a higher level of molecular structural organisation and increased Gibbs free energy. The hydration capacity is then increased in water solution, which increases the solubility of dissolved substances in the water. High-density water shows less hydrogen bond stability, higher (positive) entropy and a reduced free energy available for hydrogenation processes (21). Increased molecular organisation and lower entropy in water facilitates and makes chemical reactions more effective, requiring lower activation energy than normal (21,22,23), and giving advantageous functional effects in biological systems.

### Summary of the invention

In one aspect the present invention relates to synchronized water, in which all single water molecules at the same time are arranged in an identical way to a stable homogeneous macrostructure, wherein said synchronized water in a distilled condition and at atmospheric pressure has
a) a density of from 0.997855 to 0.998836 g/ml at 22°C,
b) a water temperature of from -6.7°C to -8.2°C at the freezing point,
c) a melting point of from 0.1°C to 0.2°C,
d) a surface tension (at 22°C) of from 72.3 to 72.7 dyn/cm, and
e) a dielectric constant of from 82.4 to 82.6 F/m.

The present invention also relates to a medium containing said synchronized water.

Further, the present invention relates in another aspect to a method for production of synchronized water, wherein light having a wavelength of 360-4000 nm is brought to pass through a topographic geometrical matrix and thereafter is brought in contact with water or a medium containing water, wherein said topographic geometrical matrix has the ability to influence the incident light in such a way that the water is synchronized and thereby is provided with the properties defined in claims 1-5.

In a further aspect the present invention relates to different medical and non-medical applications and uses of the synchronized water or the medium containing said synchronized water.

The present invention also relates to a specific topographic geometrical matrix having the properties defined below.

The present invention also relates to treatment of several of the disease conditions defined below by administration of a preparation containing the synchronized water to a human or an animal in need thereof.

Further information about the objective and the problems which are solved by the present invention appears from the following description part and the accompanying drawings, as well as from the appended non-dependent claims.

### Short Description of the Drawings

Figure 1 shows the temperature variation in control mineral water (A) and in TGM-exposed mineral water (B). Five test series were performed for each water, respectively.
Figure 2 shows the mean values (±SD) of normalized temperature variation in control mineral water (Control) and TGM-exposed mineral water (SL matrix).
Figure 3 shows the conductivity in H2 mineral water after addition of Bindzil Silica (BZA and BZB), respectively, and with TGM matrix (ELT), analysed during day 1 (M) and day 2 (M2), respectively.
Figures 4 A-C show chemical properties; pH (A), redox potential (ORP)(B), and relative hydrogen (rH)(C) in H2 mineral water after exposure during 5 minutes for controls (H2K, H2K634), as well as topographic matrixes (SphereS, SphereT, AiresP, AiresG, SQC, Hmatrix) and light at a wavelength of 634 nm and in microcluster water (Crystal Energy^{®}; CR). The analyses were performed on three occasions, i.e. directly in connection with the above-mentioned exposure (1), and after storage in a closed plastic bottle (darkness at ambient temperature) during 1 week (2) and 2 weeks (3), respectively.

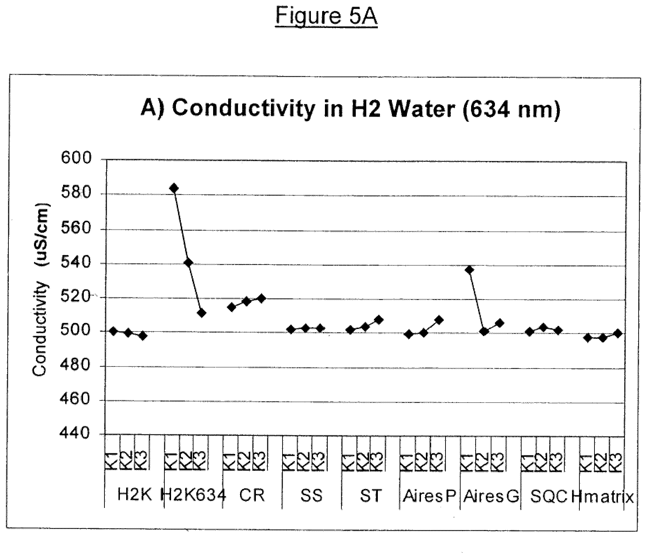

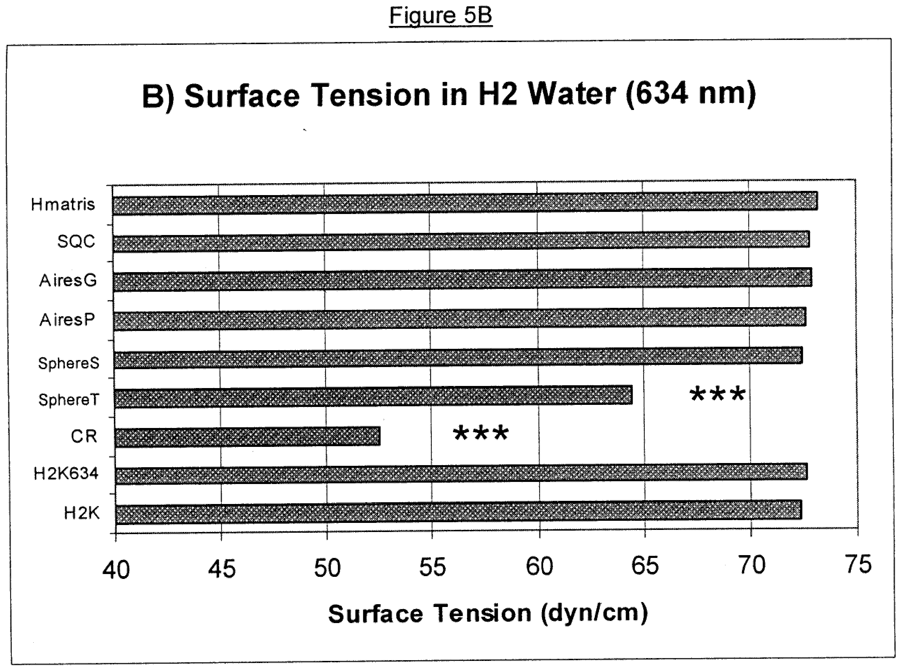

Figure 5 shows chemical properties; i.e. the conductivity (A) and surface tension (B) in H2 mineral water after exposure during 5 minutes for said controls (H2K, H2K634), and topographic matrixes (SphereS, SphereT, AiresP, AiresG, SQC, Hmatrix) and light at a wavelength of 634 nm, as well as in microcluster water (Crystal Energy^{®}; CR). The analyses were performed on three occasions, directly in connection with the above-mentioned exposure (1), and after storage in a closed plastic bottle (darkness at 23°C) during 1 week (2) and 2 weeks (3), respectively. The surface tension measurement was made two weeks after the termination of the test (n=3). *** P<0.001.
Figure 6 shows the amount of converted substrate (normalized absorbance data) as a function of the time. The trypsin activity follows a linear relationship for the control and TGM (SSc), respectively, at 0.23 mM BAEE. The slope (y = kx + l) of the curves discloses the initial rate of trypsin activity with TGM buffer and control, respectively. The dots measured in the diagram represent a mean value of 3 repetetive measurements.
Figure 7 shows the time-dose dependent (30-40 min) inhibition of the trypsin activity for control and TGM exposure (SSc), respectively. TGM buffer (SSc) reverses the trypsin activity, which is particularly notable at 30-32 min. The data measured and shown in the diagram represent a mean value ± SD of 5 repetetive measurements. * P<0.05, *** P<0.001.
Figure 8 shows the colloidal stability of untreated and treated, respectively, milk (semi-skimmed) after storage at 4°C during 21 days.
Figure 9 shows the colloidal stability of untreated and treated, respectively, ecological milk during 28 days under corresponding conditions as those shown in Figure 1, with the difference that also control milk was stored in a glass bottle.
Figure 10 shows a UV/VIS spectrum (transmittance) of a topographic matrix, SphereS (1) and SphereT (2), projected on a laser foil (polyester). K means control foil without matrix. S = black colour print and T = turquoise colour print on the matrix.
Figure 11 shows the geometrical entropy in light for selected wavelengths (412-680 nm), evaluated from graphical image analysis after exposure of transparent topographic matrixes A) Sphere (black and turquoise colour). * P<0.05, ** P<0.01.
Figure 12A shows tests performed with topographic geometrical matrixes printed on quartz glass. *** P<0.001. Abbreviations: SSsmall 1x1 mm (inner point), SSbig, SSc, 1 = 1,5 mm (inner circle diameter) and a line width of 0.1 mm. SSC2 = 2 mm circle diameter and 0.1 mm line width, respectively; SSc7 = 3 mm circle diameter and 0.1 mm line width; SSc8 = 3 mm circle diameter and 0.35 mm line width, respectively. N = 30 measurements.
Figure 12B shows tests performed with a SSc matrix and quartz: inner circle diameter - entropy G. * The dots marked represent a TGM with a line width of 0.55 mm. ∧ The dots marked represent a matrix having a line width of 0.35 mm. Non-marked dots represent a matrix having a line width of 0.1 mm.
Figure 13 shows the mean systolic (A) and diastolic (B) blood pressure of 15 healthy volunteers after consumption of 100 ml mineral water (KV) and functional water (FV), respectively.
Figure 14 shows the mean concentration values of immunoglobulin A (IgA) in unstimulated saliva in relation to control data after consumption of 100 ml mineral water and functional water, respectively, of 15 healthy volunteers. The IgA was significantly increased (P<0.009) after consumption of functional water.
Figure 15 shows power spectral density (PSD) diagrams from 10 minutes ECG registered from a woman (A) and a man (B). The figures represent the following; control (1), computer on (2), begonia water with synchronized water (3), control begonia (4), and computer on (5). Note the shifts in the scale, in particular between the diagrams 2 and 3. (VLF = very low frequencies, LF = low frequencies, and HF = high frequencies).
Figures 16A-F show different examples of topographic geometrical matrixes which are useful in the method according to the present invention.
Figure 17 shows a comparison of the precipitation kinetics for CaCO₃ between solutions exposed to SS matrix and daylight and control samples exposed to day light only.
Figures 18A-F show the variation in pH and temperature in distilled control water and synchronized water, respectively, as well as the variation in oscillating profile between pH and temperature in each type of water, and non-thermic co-oscillation as an effect of a conditioned state of the synchronized water.

### Detailed Description of the invention And Different Embodiments thereof

More precisely, the synchronized water according to the definition below may be produced by submitting water or a water-containing medium radiation with light within a certain wavelength range, wherein the light before it hits the water or the water-containing medium is brought to pass through a specifically designed topographic geometrical matrix. The design of said matrix changes the properties of the passing light in such a way that when it thereafter hits the water or the water-containing medium it creates a previously unknown synchronization of the water molecules. The properties of the synchronized water differ from those present in so-called clustered water (36-39) and similar types of water previously disclosed within the technical field. The synchronized water according to the present invention shows unique physical properties in that in a distilled condition at atmospheric pressure it has a density of 0.997855 - 0.998836 g/ml at 22°C, a water temperature at the freezing point of -6.7°C to -8.2°C, a melting point of from 0.1 to 0.2°C, a surface tension (at 22°C) of from 72.3 to 72.4 dyn/cm, and a dielectric constant of from 82.4 to 82.6 F/m.

A further unique feature of the present invention is that the synchronized water shows a non-thermal magnetic oscillation frequency range of 4-50 µHz.

A further unique feature of the synchronized water is that during exposure to daylight at room temperature during 10 h it shows an average temperature increase of at most 0.1°C, while the corresponding average temperature increase for non-synchronized water is at least 0.5°C.

The synchronized water according to the present invention is also unique in that in relation to its original non-synchronized condition under otherwise identical conditions at the same time it shows further specific properties, such as an increased conductivity, a changed pH, a reduced redox potential, a reduced relative hydrogen, and a reduced dissipative geometrical entropy.

The values and the changes measured for the above-defined parameters have been registered by measurements before and after radiation through a topographic geometrical matrix, and distinct differences have been demonstrated between the synchronized water according to the present invention and non-synchronized water under otherwise identical conditions.

The expression "under otherwise identical conditions" used herein is intended to mean that the only difference between the conditions before and after the measurements performed is the radiation and said matrix, i.e. the other conditions in and around the water-containing medium are identical.

The expression "room temperature" used herein is intended to mean a temperature of about 18-25°C, unless no other specific temperature within the interval is stated, but the same result as to the provision of synchronization is also reached at several centigrade units outside this interval.

With the expression "atmospheric pressure" used herein is intended its conventional meaning, i.e. the present surrounding air pressure at which a measurement or a test has been performed. Minor deviations in the surrounding air pressure from place to place are here intended to be comprised in the term atmospheric pressure.

The expression "daylight" used herein is intended to mean that the radiation has been performed indoors during the light hours of the day, direct sunlight however being avoided. Daylight covers the whole of the visible part of the light spectrum and thus lies in the range 360-4000 nm.

The expressions "water in distilled condition" and "distilled water" used herein are intended to mean distilled water in its conventional meaning, i.e. water which via distillation has been purified from non-volatile compounds, e.g. dissolved salts, micro-organisms and slurried substances, which otherwise are present in normal and distilled water.

The expression "water temperature at the freezing point" used herein is intended to mean the temperature to which the synchronized water has decreased when it freezes at the freezing point and then is transformed to ice having the temperature 0°C.

The expression "melting temperature" used herein is intended to mean the temperature at the phase conversion between ice and water in its liquid form.

With the expression "conditioning" used herein is intended a comprehensive term for a condition change, wherein a response is triggered by a specific stimuli leaving a permanent effect on a defined system. This term is here intended to mean essentially the whole process during which the synchronization of the water takes place. The expression "conditioned water" which occasionally is used in the application text thus means water which has been subjected to a synchronization process.

The expression "functional water" used herein is intended to mean a synchronized water to be used in biological contexts according to its meaning in view of functional food. Although the water is health stimulating, it cannot be regarded as a medicament, as medicaments are based on conventional receptor mechanisms. Instead, the functional aspect of synchronized water creates prerequisites for the body's self-regulating mechanisms to re-establish physiological homeostasis in the organism, leading to a self-healing of the body.

With the expression "a magnetically induced oscillation frequency range of 4-50 µHz" used herein is intended an oscillating co-variation of non-thermal nature in view of a chemical parameter in relation to a corresponding solar-induced temperature variation. Further details are given in Example 4.

When it is stated in the present application text that a water-containing medium is subjected to treatment with a view to synchronizing the water therein, it is also to be understood that also water as such may be treated in that way, although not explicitly stated.

Further details about the synchronization effect, the mechanisms behind this and how the synchronization of water can be measured and verified, are presented below.

Of fundamental importance for the physical aspects of water is its temperature behaviour around and at the freezing point at 0°C. At room temperature liquid water becomes denser with lower temperature. However, at 4°C water reaches its maximum density, and as the water is cooled further towards its freezing point, the liquid water expands to become less dense. The reason is related to the crystal structure of ordinary hexagonal ice. When water cools, it is structurally adapted to a crystalline hexagonal lattice configuration that stretches the rotational and vibrational aspects of the covalent bond. The effect is that the water molecules are pushed further away from neighbouring molecules. This effectively reduces the density of water when ice is formed.

The density of water is dependent on the concentration of dissolved salt as well as the temperature of the water. The salt concentration of the oceans lowers the freezing point by about 2°C and lowers the temperature of the density maximum of the water towards the freezing point.

The ability of hot water to freeze faster than cold water most likely depends on the degree of supercooling, under certain circumstances, being higher in initially cold water than in initially hot water. The initially hot water appears to freeze at a higher temperature (less supercooling) but a small part of the apparently frozen ice is solid and a considerable amount thereof constitutes entrapped liquid water. Initially cold water freezes at a lower temperature to a more solid ice with less amounts of included liquid water. The lower temperature causes intensive nucleation and a faster crystal growth rate. If the freezing temperature is kept at about -6°C, then the initially hot water is most likely to (apparently) freeze first. If the freezing is continued, the initially cold water always freezes completely, but hot water (for example, 90°C) often (but not always) appears to freeze faster than the same amount of cold water (for example, 18°C) under otherwise identical conditions.

The reason why initially cold water supercools to a higher extent is explained in terms of the gas concentration and the clustering of water. Icosahedral clusters do make difficult the necessary arrangement of water molecules to enable hexagonal ice crystal initiation; such clustering being the cause of the facile supercooling of water. Initially cold water will have a maximum (equilibrium) concentration in such icosahedral clustering. Initially hot water has lost much of its ordered clustering and, if the cooling time is sufficiently short, this will not be fully re-attained before freezing. Experiments with lowdensity water around macromolecules have shown that such clustering processes may take some time. It is also possible that dissolved gases may encourage supercooling by increasing the degree of structuring, by hydrophobic hydration, in the previously cold water in relation to the gas-reduced previously hot water (the critical effect of low concentrations of dissolved gas on the water structure has been reported, wherein re-equilibration takes several days) and by increasing the pressure when the gas comes out of the solution as the water starts to crystallize, thereby lowering the melting point and reducing the tendency to freeze. Also, the presence of tiny gas bubbles (cavities produced on heating) may increase the rate of nucleation, thereby reducing the supercooling.

The dielectric constant (permittivity) of water is a physical quantity that defines how an electric field affects and is affected by the dielectric medium, i.e. water, and is determined by the ability of water to polarize in response to the field, thereby reducing the total electric field in the water. Thus, the dieleotric constant relates to the water's ability to transmit (or "permit") an electric field. Dependent on the frequency, the dipole (water molecule) may move with time in relation to the field, lag behind it or remain apparently unaffected. The ease of the movement depends on the viscosity and the mobility of the electron clouds. In water this, in turn, depends on the strength and extent of the hydrogen bonded network. In free liquid water the movement occurs at GHz frequencies (microwaves), whereas in more restricted "bound" water it occurs at MHz frequencies. At a frequency range of 40-50 MHz the dielectric constant of distilled water is 80 F/m at room temperature.

The cohesive forces between the molecules in a liquid, e.g. water, are shared with all neighbouring atoms. Those on the liquid surface have no neighbouring atoms above, and exhibit stronger attractive forces upon their nearest neighbours on the surface. This enhancement of intermolecular attractive forces on the surface is called surface tension.

The expression "topographic geometrical matrix", in the following sometimes abbreviated "TGM", used throughout the application text is intended to mean that the design of the matrix in question is based on classical geometry created from interference between standing waves having fractal properties.

The expression "topographic" used throughout the application text is intended to mean a dynamic or variable geometrical form or structure in 2D or 3D format.

The expression classical geometry used throughout the application text is intended to mean a materialisation in the form of physical geometric pattems created by interaction between standing wave phenomenons of sound or light having different frequencies (sinus waves) in a medium, in which the vibration or the wave motion is manifested in structure and form (e.g. via a vibrating plate sprinkled with sand, wherein the sand, alternatively in spherical water droplets containing fine particles, self-regulatively creates standing waves with the basis on the frequency applied and with a definable geometrical structure and form.

The term standing wave is intended to mean a wave phenomenon produced by two wave motions moving in opposite directions and superposeing each other. Thereby bellies and nodes occur along the waves, as well as a wave which seems to stand still and just oscillate back and forth, i.e. a standing wave. The highest amplitude of the wave is present in the bellies and the smallest is present in the nodes, and the distance between the nodes is half a wavelength.

A standing wave in an air column is created by reflecting a pressure wave forward and backward in the ends of a cavity. These ends then constitute nodes, and a standing wave is created between them. If energy is applied in a convenient way and at a convenient location, this process may be maintained in such a way that a resonance tune arises in the cavity, i.e. a resonant standing wave. The frequency of the tone is dependent on the distribution rate, which is a physical property of the medium in which the wave moves, and the distance between the nodes. Also overtunes, multiples of the resonance tone, may be maintained by the same process.

The expression "fractal proportionality" used throughout the application text is intended to mean the presence of infinitely repeatable self-like structural elements, which by self-organisation spontaneously create a geometrical structure and form (e.g. formation of planar ice crystals).

The expression "matrix" is intended to mean the article or object which the light is brought to hit and pass before hitting the water or the water-containing medium in which the water molecules are to be synchronized. Optionally, the matrix may be arranged on a support being in direct contact with the water-containing medium or be present at a certain distance from this.

The above-mentioned matrix may in one embodiment be defined by its two-dimensional appearance in a plane which is perpendicular or mainly perpendicular in relation to the radiation direction of the light. The expression "two-dimensional appearance" is here intended to more precisely mean the two-dimensional pattern the matrix forms when viewed from the radiation source. Thus, in this embodiment the matrix may have a thickness or a depth which is considerably small in relation to its extension in the two-dimensional plane perpendicular to the radiation direction. In other embodiments the matrix may be defined by its three-dimensional appearance, such as in cases in which it constitutes a more pronounced three-dimensional geometrical figure, e.g. when the above-mentioned thickness or depth is higher and intended to influence, to a higher extent, the modification of the properties of the incident light

The support on which the matrix, in particular in its two-dimensional form, may be arranged may be manufactured from any suitable material which does not influence the electromagnetic properties of the incident light, but is preferably transparent. The support may be manufactured from glass, such as boron silicate glass (optical cover glass) or quartz glass (optical), plastic, cardboard, sheet metal, natural material or any other transparent material, such as laminates or foils.

The support for the matrix may have the form of a platform, a plate, a foil, a pipe, a spool, a wall of a storage vessel, such as a beaker, a package or a tank for the water-containing medium, etc. The matrix may be arranged on the support in any known way, e.g. in such a way that it has been plated, imprinted, glued, painted, taped, cast or laminated. In one embodiment the matrix has been imprinted on quartz glass or has been laminated.

The fields and lines present on the matrix may also have a certain spectral colour or may be a metal foil for advantageous influence on the modification of the properties of the incident light. Convenient colours/metal foils for this purpose are gold, silver, copper, black, green, turquoise, red or other spectral colours.

The support as such should, as mentioned above, not essentially influence the electromagnetic properties of the incident light. Instead, it is the design (the two-dimensional or three-dimensional design), i.e. the topographic geometrical properties, which are intended to modify the properties of the incident light with a view to obtaining the synchronized water with its unique properties.

A person skilled in the art recognizes that there is no defined limit between a two-dimensional matrix according to the definition above and a three-dimensional matrix, as the two-dimensional matrix always has a certain extension in the depth, but the present invention is intended to cover all embodiments of the topographic geometrical matrixes defined above and described below.

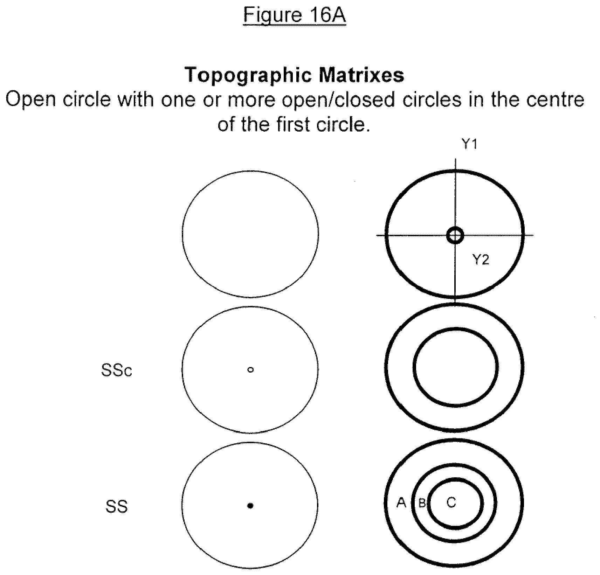

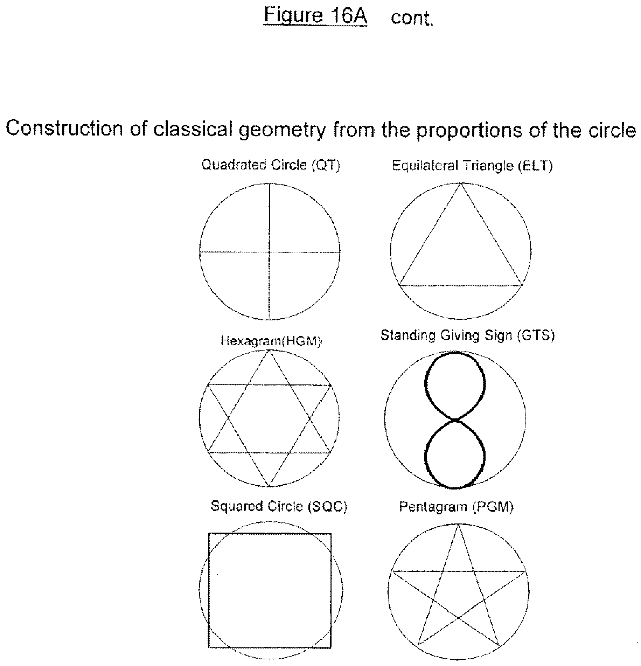

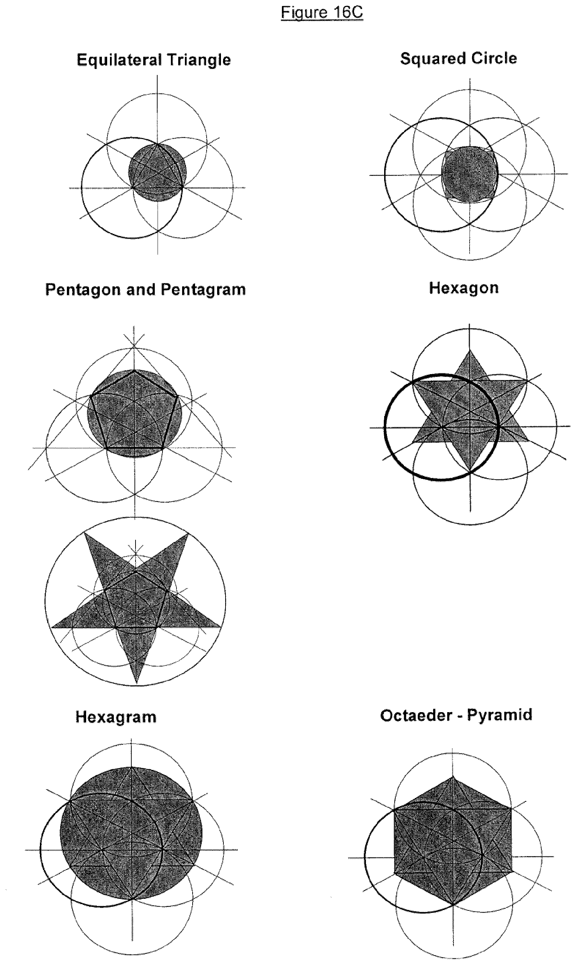

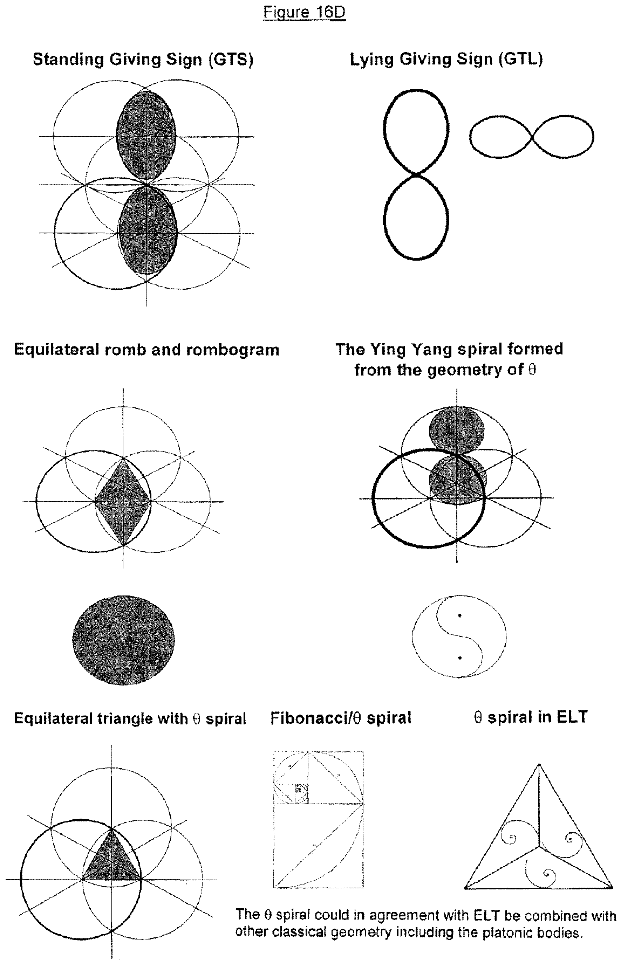

The design of the topographic geometrical matrix according to the present invention, below sometimes only called "TGM" or "the matrix", has a substantial influence on the modification of the properties of the incident light and thus provides the synchronization of the water-containing medium. Important results are particularly obtained with geometrical designs which are based on the geometry of the circle and the sphere, i.e. in view of a standing wave with fractal proportionality according to the definitions above (see also the examples in Figures 16 A-F), in which the abbreviations for each matrix are also stated.

As to the matrix viewed in the two-dimensional plane, which normally is essentially perpendicular or is essentially perpendicular in relation to the direction of the incident light, the following applies. The most simple embodiment of the matrix is an ordinary circle. Other embodiments include a circle enclosing one or more concentric circles having a common centre or a common tangential point on the arc, or a circle containing a smaller closed circle. The expression "closed" is intended to mean that the incident light not is able to pass through the closed surface. Another embodiment includes a circle containing several concentric circles, wherein one or more of the rings formed are closed. As to the embodiments with concentric circles described above synchronization is obtained at a specific relationship between different parameters of the circles. The relationship θ (phi) between the diameter of the outermost circle and the next circle counting inwards toward the common centre of the circles should be 1.68 or 1/0 or should follow Fibonacchi's sequence of numbers (40), wherein fₙ = θⁿ/5^{0.5} (0,1, 1, 2, 3, 5, 8, 13, 21, 34...) or common logarithms thereof. Thus, the same relationship should apply between the diameter of the second outermost circle in the matrix and the next circle counting inwards toward the common centre of the circles, etc. The above-mentioned relationship θ also applies to the perimeters of the above-mentioned circles.

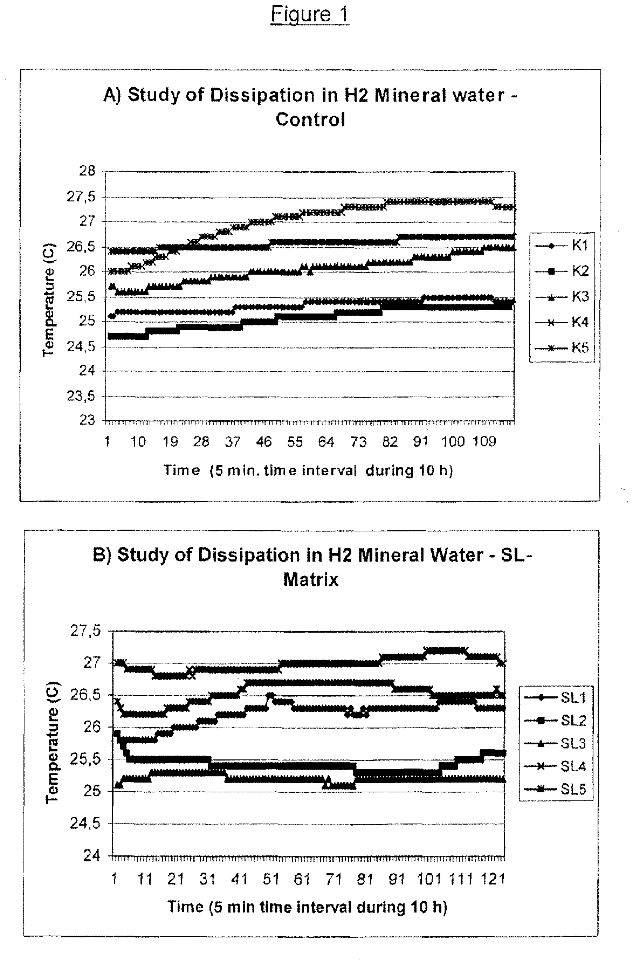

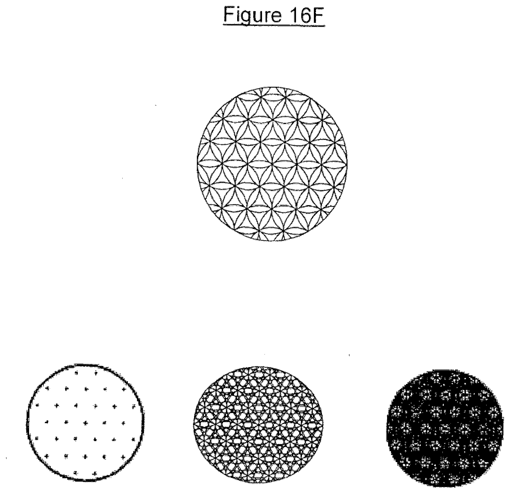

In a particularly preferred embodiment the topographic geometrical matrix has the form of an open circle concentrically enclosing a smaller closed circle (see the third uppermost variant to the left in Figure 16A, also designated SS). The diameters and line widths of the outer and inner circle, respectively, follow the relationship f (phi); A/B = B/C = 1.618 or 1/f or the abovementioned Fibonacchi's sequence of numbers, wherein A, B and C are circle diameters, areas or perimeters. In the same way the position of the inner circle follows the vertical axis Y1 - Y2 in Figure 16A, f, 1/f or fₙ. The diameter of the outer circle may vary between 2 nm and 987 µm, the inner circle diameter may vary between 0.125 nm and 233 µm, and the outer line width may vary between 0.125 nm and 8 µm, wherein, however, the above-mentioned geometrical relationships always apply.

In a concrete and preferred embodiment of the preferred SS matrix, the outer circle diameter is 13 nm and the inner circle diameter is 1 nm, said outer circle having a line width of 0.07 nm, or all corresponding measures are expressed in µm. In another embodiment the outer circle diameter is 55 nm and the inner circle diameter 8 nm, the outer circle having a line width of 2 nm, or the corresponding measures are expressed in µm.

Particularly preferred results have been obtained by use of the above-mentioned SS matrix in nanometer dimensions. The fact that better results are obtained the smaller the matrix is due to interference properties between the incident light and the matrix. The extension of the interference pattern in the medium increases with the reduced geometrical dimension of the matrix.

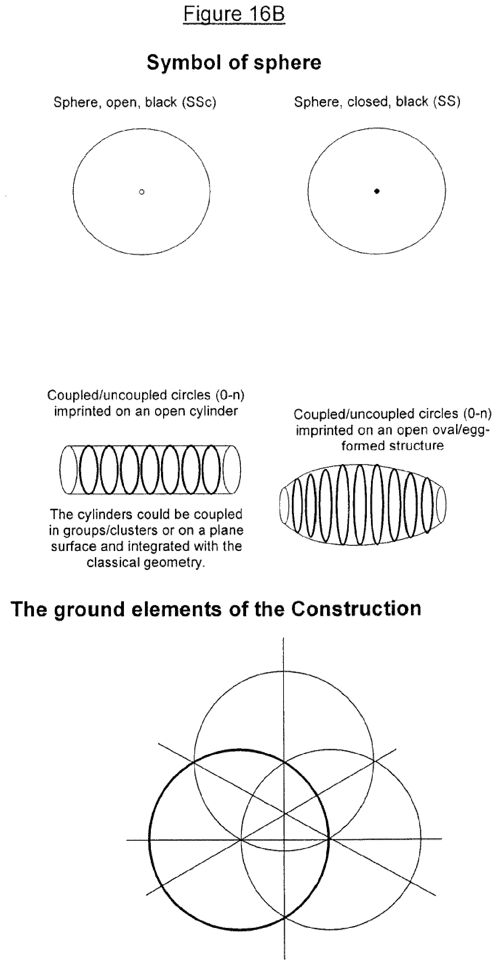

Other embodiments are those in which classical geometrical figures are present within one or more circles in a matrix. Examples of such matrixes are a quadrated circle (QT), en equilateral triangle (ELT), a hexagram (HGM), a standing (alternatively lying) giving sign (GTS), a squared circle (SQC) and a pentagram (PGM). These embodiments of the matrix may also be included in one or more of the concentric circles in the embodiments described above. In other embodiments of the essentially two-dimensionally designed matrix two or more overlapping circles having identical diameters are present. Such embodiments may be constructed by first drawing a straight line starting from the centre of the first circle (at the bottom of Figure 16B). A second circle is then constructed having its centre on the same line, the arc of the second circle intersecting the centre of the first circle. The centre of a third circle intersects the intersection of both first circles, and the arc thereof is tangent to the centre of the first and the second circle, respectively. This construction is also based on classical geometry and mathematical processes related to θ (phi) and Fibonacchi's sequence of numbers. Examples of matrixes constructed in this way are an equilateral triangle, a quadrated circle, a pentagram, a pentagon, a hexagon, a standing/lying giving sign, an equilateral rhomb and a logarithmic spiral formed from the geometry of θ (phi).

In other effective embodiments the matrix is designed according to a so-called Fibonacchi/θ spiral, and this may be present in combination with other classical geometries as stated above, e.g. in an equilateral triangle.

Other more complex embodiments of the topographic geometrical matrix according to the present invention is a triangulated hexagon, a triangulated hexagram, a star tetraeder, and a so-called Metatron cube (containing the geometry of "the 5 so-called platonic bodies" (the octaeder, the tetraeder, the dodecaeder, the cube and the icosaeder)).

Thus, synchronization is obtained when the matrix in the substantially two-dimensional plane contains classical geometrical figures as matrixes with the basis of the geometry of the circle. However, the design of the matrix appearance may slightly deviate from the classical geometry. The circle the geometry of which the appearance of the matrixes is based on may be e.g. slightly oval, and in one embodiment the circles do not have to be perfectly concentric and need not have a common geometrical centre. Circles of different sizes may also overlap each other. Thus, all of the above described matrixes may slightly deviate from the above-mentioned forms. However, the deviation may only be such that the modification of the incident light for the synchronization of the water in the water-containing medium nevertheless takes place. Thus, all topographic geometrical matrixes of the type disclosed above, and also variants and combinations thereof, are intended to be included in the scope of the present invention, as long as they have the ability to influence the incident light according to the definition below in such a way that the water in the water-containing medium is synchronized and thereby obtains the above-mentioned unique properties.

As to the embodiments of the topographic geometrical matrix which have a more pronounced three-dimensional design, these are also designed on the basis of interference induced geometry.

Different spherical embodiments of the three-dimensional matrixes described herein correspond to those for the circle in the substantially two-dimensional plane, as described above. Examples of simple three-dimensional embodiments are a simple sphere, a sphere containing a smaller open or closed sphere, a sphere containing one or more concentric spheres having a common centre or a common tangential point on the surface of the sphere. Other embodiments include three-dimensional embodiments of classical geometrical bodies such as a cube, an octaeder, a rhombogram, and other variants corresponding to those of the two-dimensional embodiments diescribed above. In embodiments which are based on the geometry of the cylinder, wherein the light is intended to hit the base surface of the cylinder, a number of so-called uncoupled circles may e.g. be arranged on the cylinder surface. The distance between the circles and the lines, respectively, in the spiral is not critical, but lies in practise within the interval which is defined by the circle geometry proportions and which separates neighbouring circles, and is not necessarily not less than or equal to the wavelength of the light. However, several similar or different cylinders may be connected in groups. In other embodiments the cylindrical form may deviate and have a concave or convex appearance, or it may be designed like an egg having truncated ends (see Figure 16B), on the surfaces of which circles may be arranged in a similar way as described above for the pure cylindrical embodiment. Also for the three-dimensional matrixes simple geometrical embodiments give satisfactory results, but, like for the above described substantially two-dimensional variants, deviations of all the matrix forms described above may be present as long as they have the ability to influence the incident light according to the definition below in such a way that the water in the water-containing medium is synchronized and thereby obtains the unique properties defined in claims 1-5.

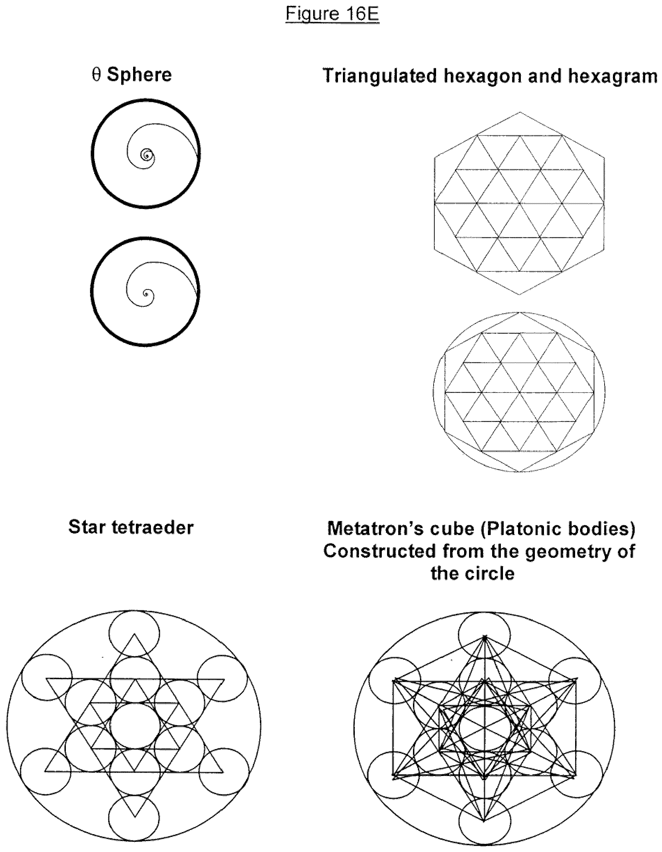

The pattern in Figure 16F is an extension of what has been called the "flower of life", i.e. a set of circles centered at hexagonal grid points, wherein the radius of each circle is equal to the grid point distance (see Figures 16B and 16E), and wherein a total of four concentric circles are drawn at each grid point with radii of 1, 2, 3 and 4 times the grid point distance.

In the examples below tests are also described performed on topographic geometrical matrixes in different embodiments, as described above and in Figures 16A-16F. The topographic geometrical matrix used in the method according to the present invention may in one embodiment as such constitute the geometrical figure without being arranged on any support. In such an embodiment the matrix may e.g. be present in the air, be suspended or arranged in any other convenient way at a convenient distance from the surface of the water or the water-containing medium to be irradiated, or at a convenient distance from the steam present in the air in the space in which the synchronization of water is to take place.

The outer diameters of the circles and the spheres, respectively, and the maximum distances between opposite ends of the matrixes described above may in practise vary between nanometer scale and up to several meters. However, optimal results are obtained in the size range of 0.1 nm and up to the µm range, such as 900 µm, with a dissolution of 10 nm. These distances are governed, as mentioned above, by the interference-creating geometry of the construction. Thus, it is important that the line width be proportional to e.g. the circle dimension. If the outer circle has a diameter of 13 mm and the inner circle a diameter of 1 mm, then the line width, based on the golden section aspects as discussed above, is more precisely 1 mm113, i.e. 0.076 mm.

The width of each line in the topographic geometrical matrixes discussed above also has a certain influence on the modification of the incident light. Satisfactory results as to the synchronization of the water-containing medium is obtained, as mentioned above, when each line has a width of from nm level to mm level, e.g. a width of 0.01-1.0 mm, such as 0.1-0.5 mm. Different lines in one and the same topographic geometrical matrix may also have different widths, wherein the outer circle e.g. may have a line width of 0.5 mm and the inner circle a line width of 0.1 mm. The useful line width increases proportionally in relation to the increase of the circle diameter, i.e. is connected to the circle geometry according to the above-mentioned Fibonacchi's sequence of numbers.

The topographic geometrical matrix according to the present invention is normally arranged in such a way that the incident light from the light source hits the present two-dimensional matrix perpendicularly in relation to the radiation direction, but synchronization may also be obtained at a rotation angle of up to 180° (cf daylight). In embodiments with matrixes of more pronounced three-dimensional character, e.g. spheres, the radiation may incide from any direction in relation to the matrix. For cylindrical matrixes and variants thereof the light may incide perpendicularly in relation to the base of the cylindrical matrix, but also at another angle of incidence of at most 360° angle of rotation (cf daylight).

In one embodiment a certain distance may be present between the topographic geometrical matrix, or the support on which this is arranged, and the surface of the water-containing medium (or the water). In this embodiment the space between the matrix and the water-containing medium contains air. Further, the incident light direction is normally perpendicular in relation to the surface of the water-containing medium, or the angle of incidence may deviate by up to 180°. Said distance is not critical and may be long, but in several embodiments the matrix and/or the support on which it is arranged is/are in direct contact with the water-containing medium and may also be partially suspended in the water-containing medium.

The light source for the light is not critical and may be any one of known light sources within the technical field for radiation of light within the wavelength range in question.

Examples of useful light sources are a spectrophotometer, daylight, full-light lamps, diodes, spectral filters, etc.

The light used in the present invention is either continuous or pulsing, wherein in the latter case it may consist of only one single pulse or several different pulses.

The wavelength of the light used lies in the range 300-4000 nm. For daylight it could be noted that daylight besides electromagnetism also contains a solar magnetic field, which is further described in Example 4 below. In one embodiment the wavelength lies in the range 360-900 nm. Synchronization has been obtained in the range for visible light and the lower IR (infred) range, and e.g. a wavelength of 634 nm has been useful according to the present invention.

When the incident light from the light source is made to pass the topographic geometrical matrix, its character is changed in such a way that the geometrical entropy in the spectral electromagnetic light is changed in view of its spatial form and field structure, wherein an increased coordination of single wave components of both electric and magnetic nature leads to a "laser-like" coherent self-stabilizing light. These amendments may be measured on spectral light (e.g. 634 nm) from an ordinary light bulb in a spectrophotometer emitting out a non-coherent light. The amendments in the physical light properties after passage of the matrix are registered with a highly sensitive video camera, whereupon the image is analyzed and evaluated mathematically by optical spectral imaging.

In one embodiment, the distance between the light source (which may also be daylight) and the topographic geometrical matrix is 2-25 cm, preferably 2-10 cm.

In one embodiment one or more light sources emitting light of different wavelengths within the present wavelength range may be used in combination.

The expression "water-containing medium" used throughout the application text is intended to mean any medium containing any form of water, e.g. a solution, a slurry, a suspension, a body fluid, a paste, a semi-solid substance, a solid formulation or a solid, and air containing steam etc, which is useful within any technical field, e.g. the pharmaceutical field, the food area, technical microbiology, the climate and ventilation field and the lens cleaner fluid field.

Examples of foods which can constitute the water-containing medium are beverages, such as table water, functional waters and foods, juices, milk and other dairy products, bakery products, fruit and vegetables, etc. Also deep-frozen foods are intended to be covered by the expression "water-containing medium". Thus, any food containing water may be treated with the method according to the present invention.

The water content in the water-containing medium is not critical, but varies normally between 60 and 100 vol%, preferably 80-100 vol%.

The water to be synchronized may by definition consist of normal water in various forms, such as salt water, drinking water, fresh water, mineral water, distilled or deionized water and other types of water-containing differrent amounts of different natural and artificial constituents, such as dissolved salts, etc.

In the method according to the present invention the water-containing medium or the water may be present in any open or closed container, such as beakers, containers, tubes and tanks of different volumes. The volume of the water-containing medium is not critical, but normally volumes of up to 100 m³ may be treated. The volume in question is of course dependent on the final application field, but for practical reasons the treated volume of the water-containing medium is normally 1-100 m³, or alternatively 0.1-2.0 I. The relationship between the surface area of the water-containing medium hit by the incident beams and the volume of the water-containing medium is also not critical, and a synchronization effect is obtained even if the beams only reach a part of the water-containing medium.

Further, the water-containing medium may stand still or be in motion in the method according to the present invention, such as during flow or vortex treatment. Thus, the water-containing medium to be radiated in the method according to the present invention may pass the light source on e.g. a conveyor belt in an industrial manufacturing process. Alternatively, the light source and the matrix may be moved during the radiation, and the water-containing medium may be stationary. The temperature of the water-containing medium is also not critical and may vary from the freezing temperature up to the boiling temperature. This also applies during the use of the treated water-containing medium containing synchronized water.

The time period during which the water-containing medium is subjected to matrix-modified radiation in the method according to the present invention is not critical, but is normally from some seconds to permanent exposure, e.g. 1 min.

The duration of the synchronized condition of the water in the treated product is permanent, unless the product is subjected to outer disturbances, e.g. agitation, shaking or exposure to powerful electromagnetic fields. If the synchronized condition would be broken due to any outer disturbance, a spontaneous return to the synchronized condition will soon take place.

The stability of the synchronized water in the radiated water-containing medium may be extended and increased if an object made of quartz (here alternately called silica) is present in the medium, e.g. in the form of a crystal, a plate, spheres, particles and colloids having a diameter of from some centimeters down to 1 nm. As to colloidal media the colloid diameter may be 1-5 nm and the concentration 0.1-100 µg/ml. This silica may be present in the water-containing medium even before the radiation, or alternatively it may be added during or after the radiation process with a view to prolonging and increasing the stability of the product containing synchronized water.

Further, the synchronized nature of the water may be transferred to an untreated water-containing medium due to the self-regulating effect. A liquid containing synchronized water may e.g. be mixed with another water-containing medium, wherein the synchronized properties are transferred in such a way that all the water in the mixture is synchronized. Thus, the radiated water-containing medium which contains the synchronized water produced according to the method of the present invention may be further processed to its final form before the final use, provided that the preparation steps do not include any measures that would abolish the nature of the synchronized water. Under stable conditions the treated medium or the final product containing synchronized water may be stored for a long time, such as up to at least 3-5 years, without abolishing the synchronized nature of the water.

When a water-containing medium is radiated in the method according to the present invention, the water molecules first hit by the incident light are synchronized. Thereafter, the synchronization is spread automatically to all water molecules in the medium until all the water molecules in the medium are synchronized. The synchronization effect takes place instantaneously and at the speed of light in both liquid and air.

The "synchronized" water obtained with the method according to the present invention is intended to mean, in addition to what has been defined above and in the subsequent claims, that two or more related events take place simultaneously in the water, wherein co-ordination between the oscillations in oscillators in the form of single water molecules takes place with spontaneous order and rhythm. Induction of synchronization in water means that the water assumes a condition of spontaneous self-similarity, in which each water molecule shows an oscillating condition which is identical with the condition of any other water molecule in the medium. The water shows molecular co-ordination and co-operativity and acts like a macroscopic infinite molecule, which reduces the entropy, increases the Gibb's free energy and delocalizes the access to surface-active electrons, which as mentioned above, inter alia results in increased density, lower freezing point, higher melting point, increased dielectric constant, reduced surface tension, non-termic oscillation of pH, amended pH, increased conductivity, reduced redox-potential and reduced relative hydrogen. A dissipative entropy change, followed by a temperature decrease feeds back to a self-regulatory condition of a thermodynamic equilibrium. The phase shift in the water creates a coherent, well-ordered microscopic field, in which molecular integration reduces the normal electric resistivity of water and therefore increases the conductivity. The presence of colloidal quartz potentiates the liberation of free energy in synchronized water. The self-regulative molecular co-operativity of the synchronized water further reduces the surface tension and increases the wettability of the water, wherein less energy is required with a view to maintaining substances in solution. It should also be noted that once synchronization takes place, it is complete, i.e. no partially synchronized water exists.

The difference between conventionally clustered water and synchronized water is the following: the clustered water in the meaning as it is known within the technical field (2, 8, 9, 10, 11, and 36-39) is characterized by the presence of macroscopical permanent interactive water clusters having a limited voluminous size (LDV and HDV clusters, respectively) in a permanent exchange and reorganisation of single water molecule's localisation and migration as well as by instantaneous dissolution and re-formation of single hydrogen bonds. Thus, clustered water is conventionally described from a classical dynamic perspective of chemical bonding.

According to the Swedish National Encyclopaedia (Vol. 19, 1996, pages 280-281), e.g. a cluster model is described as an instantaneous arrangement in water with a duration of about one nanosecond, wherein each water molecule in these clusters is bound to three to four other water molecules, while the molecules outside are non-bound. The arrangement and the boundary of the clusters are changing all the time by co-ordinated motion of the water molecules. However, the synchronized water according to the present invention is to be regarded as a self-like co-operative system defined from an energetic perspective. The physical and chemical properties defined above ambiguously describe the presence of a coherent self-stabilizing synchronized water characterized by a stabilized geometry, dissipative properties, co-operative synergism and increased free energy, wherein the water acts as a macromolecular liquid or fluid crystal having uniform properties.

The synchronized water according to the present invention may be distinguished from normal, non-synchronized water in several ways. The experiments performed in the examples below show that synchronized water differs from other types of water, both in physical and in chemical aspect.

Thus, Example 1 shows a change in the water density at 22°C, a lower water temperature at the freezing point, a higher melting point, a lower surface tension, and a lower dielectric constant. Figure 1 shows a time-dependent temperature reduction including increased temperature stability in water at a spontaneous temperature increase in the surrounding environment Example 3 shows a time-dependent conductivity increase after the addition of colloidal quartz. Examples 2 and 3 show that the change in temperature and conductivity, respectively, is induced due to spontaneous, self-regulatorily operated energy processes, which is typical of the synchronized water according to the present invention. Example 4 shows a non-termic oscillation of the pH in synchronized water, wherein a unique frequency pattern occurs in the range of 4-50 µHz.

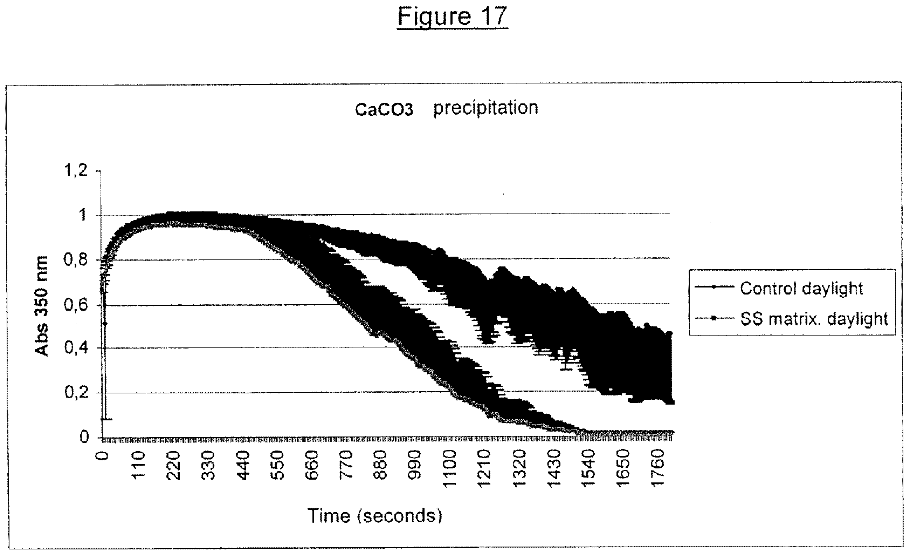

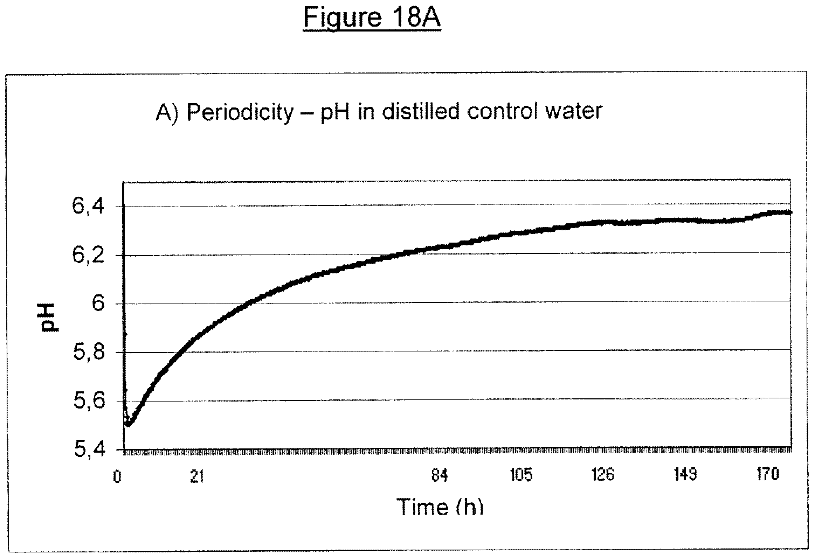

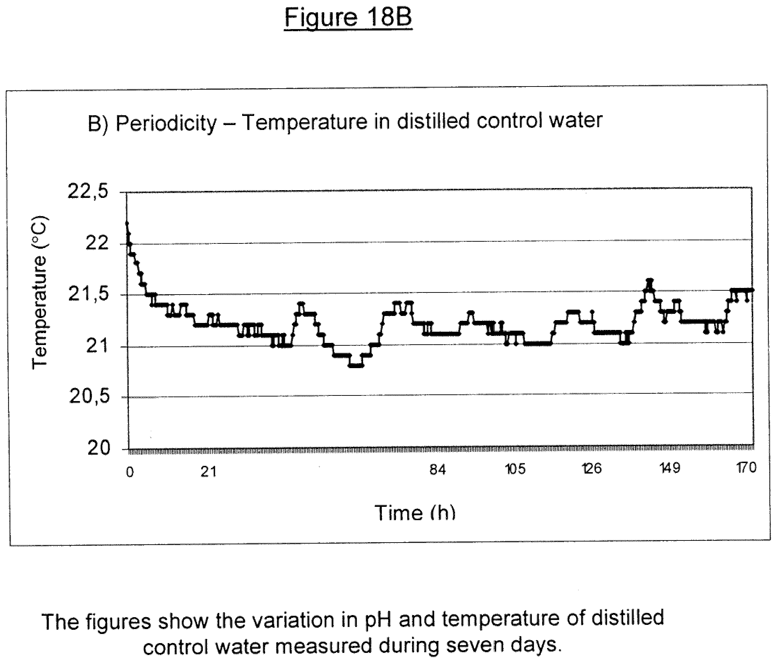

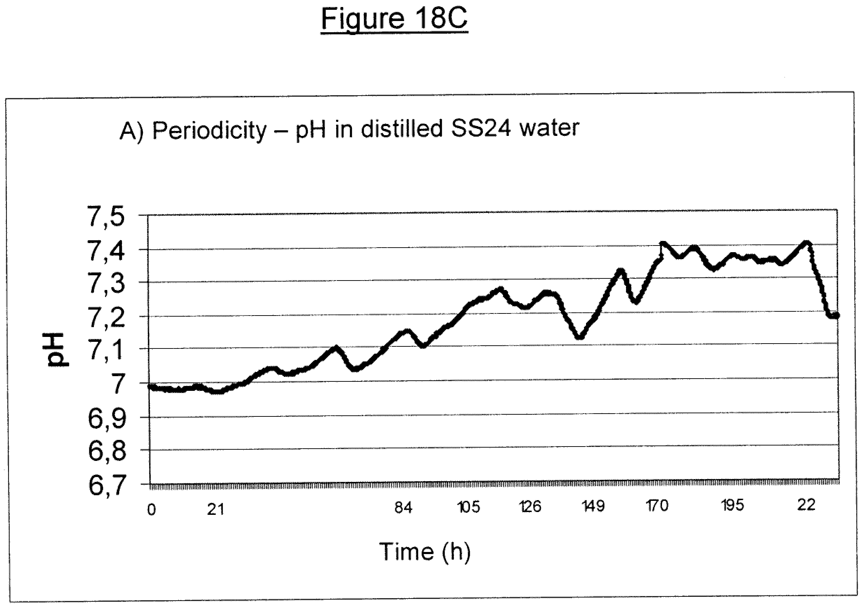

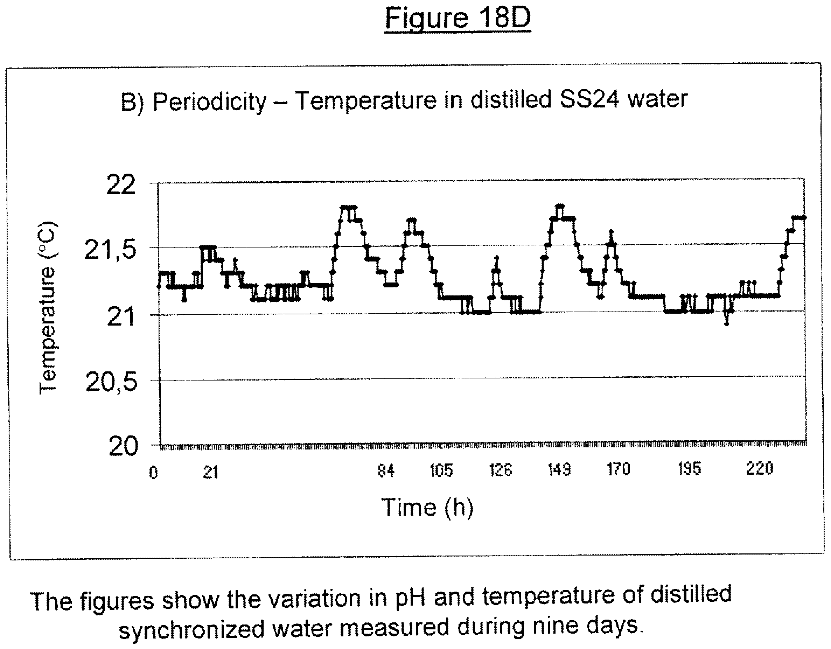

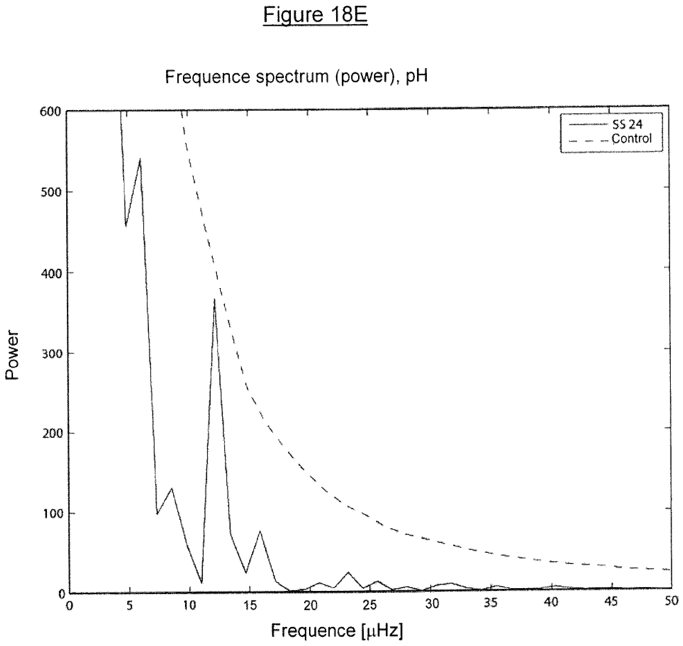

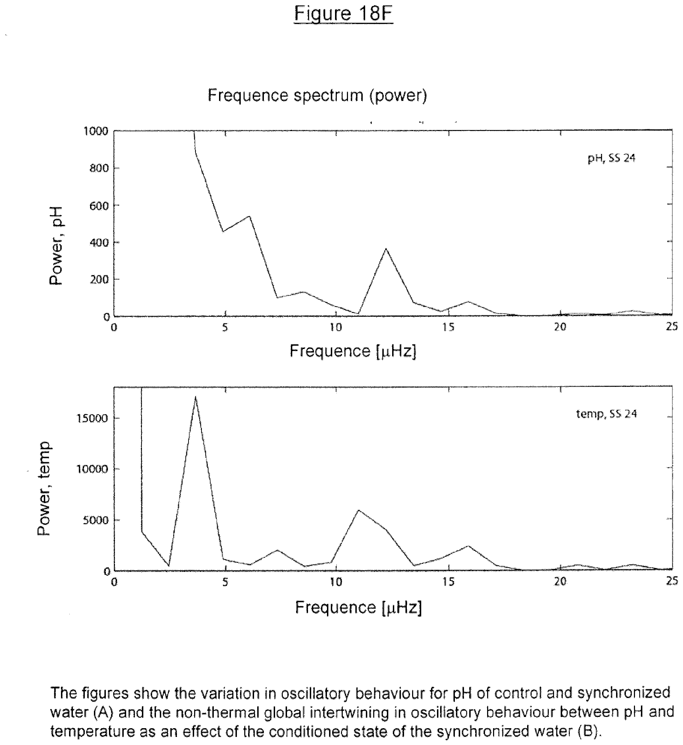

The mechanism behind the synchronization of water is not fully elucidated. A model which at least partly could explain the mechanism is the change of the electromagnetic/magnetic components of the light during the passage of the topographic geometrical matrix, which is described in more detail in Example 12 with reference to Figure 17, and via Fourier transformation analysis in Example 4 and Figure 18.

There is also a potential possibility that the synchronized water also influences the presence of ¹⁷O in water, which may be indicated by NMR analysis.

The Belousov-Zhabotiskii reaction (the B-Z reaction) creates a three-dimensional dynamic wave form of a torus geometry in aqueous sulphuric acid solution containing malonic acid, sodium bromate and terrain as the redox indicator (30). When the water is activated, standing scroll waves are formed, which dynamically roll over the water surface driven by a circularly expanding oxidation/reduction of ferroin. The system is self-regulating and forms pared counter-rotating spirals. Also here there is a potential possibility that the B-Z reaction is initiated by synchronized water.

### Example 1

The density, the dielectric constant, the surface tension and the temperature profile at the freezing and melting point were examined in synchronized distilled water. Distilled water (Apoteket AB, Sweden) was exposed to daylight and a TGM matrix for 24 h at ambient temperature. The temperature characteristics were followed with NiCrNi sensors via collection (Temperaturlogger, Nordtec AB, Sweden) of temperature data every third second during up to 8 h. The density of the synchronized water was analysed by balancing (Mettler, GTF, Sweden) in a known volume of water. The dielectric constant of water was analyzed with a Percometer (Adek Ltd, Estonia). The dielectric probe was shielded in a Faraday's cage.

The properties of synchronized water after the TGM conditioning are listed in Table 1 below.

**Table 1**

| Parameter | Matrix | | | |
|---|---|---|---|---|
| | Reference | SS | SSc | GTS |
| Density (g/ml)# | 0,997800 | 0,996246 ± 0,000098*** | 0,998133 ± 0,000278** | 0,998410 ± 0,000426** |
| Permittivity. Faraday box ((F/m)# | 80 (77,7^) | 82.5 ± 0,1* | - | - |
| Water temperature at the Freezing point (°C)* | 0 | '-6,7 ± 0,8*** | '-8,2 ± 0,3*** | *-7,6 t 1,3*** |
| Melting point (°C)* | 0 | '0,2 ± 0,01* | '0,1 ± 0,00* | '0,1 ± 0,05* |
| Surface Tension (dyn/cm)# | 73 (72,9^) | 72,3 ± 0,02*** | 72,7 ± 0,02** | 72,7 ± 0,02** |

| | | | | |
|---|---|---|---|---|
| ⁻ The characterization is related to conditioning of distilled water for 24 h with either of the following TGM (SS, SSc, GTS) and exposed for daylight:. * All values are the mean (± SD) of two repetitive analyses during three consequtive days. # Values are the mean of thirty repetitive measurements analysed on three occations at 22°C. ^ Experimental reference value. * P<0,05; ** P<0,01; *** P<0.01 | | | | |

The synchronized water was found to have a substantially higher density at ambient temperature (22°C) after TMG treatment. The relative density calculated on the basis of the average of measured densities after TGM condition varies between 0.997855 and 0.998836 (P<0.01-0.01).

The average water temperature at the freezing point in synchronized water varies between -6.7°C and -8.2°C (P<0.001). The corresponding melting point range was 0.1-0.2°C (P<0.05). The dielectric constant during the TGM treatment was substantially increased and was 82.4-82.6 F/m (P<0.001). The surface tension was substantially reduced after the TGM treatment, in particular with the SS matrix (72.3 dyn/cm (P<0.001)) and the average was in the range of 72.3-72.7 dyn/cm.

The reason why slightly different values for the synchronized water were measured during use of different matrixes is that the synchronization may be gradually specifically and selectively pronounced due to the fact that matrixes with different geometry create a selective difference in corresponding interference patterns with incident electromagnetism, wherein formations arise in the water which manifest in a unique profile as to physical and chemical parameters, thus somewhat deviating between different matrixes. However, the difference due to the conditioning between synchronized and non-synchronized water is so pronounced (see Table 1) that there is no doubt whether a water is synchronized or not. All differences measured in the parameters described are profiled and changed in a similar way, which nevertheless is unique for each interference pattern.

To conclude, the result indicates that in distilled synchronized water the binding and the order of water structures is organized to a great extent. The density increase indicates formation of a "flowing crystal structure" which differs from the ordinary hexagon-like structure present in cold water and ice. As the network configuration during the experiment is present and maintained at room temperature, which in a high degree differs from the ordinary hexagonal order at the freezing point, the water with the inherent synchronization during the experiment aims at a self-regulating molecular feedback by the formation of tetrahedal modular water structures for the construction of a continuous water having a high structural symmetry regulated by hydrogen bonds between stabilizing "real" water structures. The several directed hydrogen bonds in synchronized water introduce a highly stable inter- and intramolecularly bound self-organizing biosystem.

The reduction of the water temperature to below zero before the freezing takes place supports the formation of a system organizing water structures stabilized by the hydrogen bonds. A change of the morphology in synchronized water requires time before the shift to the hexagonal network configuration at the freezing point. The marginally higher melting point of the synchronized water in the form of ice indicates re-formation of the self-organizing water structures at a temperature above the melting point of the non-synchronized water.

The increase in the dielectric constant changes the character of the hydrogen bond, i.e. both the strength and the extent of hydrogen bonding increases. This 1) makes the mobility of the molecular water dipole difficult and restricts the ability of the water molecule to oscillate at a higher frequency, 2) increases the inertia in the rotation of the water molecules, i.e. increases the friction and thus the dielectric loss, and 3) changes the ordinary water structure.

The reduction in surface tension makes the conditioned synchronized water more wet and more fluid, which supports the co-operativity and the adjacent dynamic mobility and the fluidality in the close adaptability between neighbouring water structure modules, i.e. a condition which increases the water density.

To conclude, the changes in the studied physical properties support the fact that synchronized water to a higher extent is self-organized based on the formation of a modular interacting system consisting of co-organizing water structures.

### Example 2

Dissipative systems' character of order, synchronicity, lower entropy and temperature may be illustrated with a longitudinal natural water flow passing an obstacle in the form of e.g. a stone, which is centrally located in the water flow. Downstream the stone the water forms laminar vortex streams which spontaneously create lateral concentration and temperature gradients (24). The temperature of the water decreases downstream the stone by 0.1-0.4°C. When synchronized organized water hits the obstacle, a condition of temporary organizational chaos arises. The longitudinal vortex of the water collapses, and the energy is emitted to the surroundings and the temperature increases. Downstream the stone the laminar vortex motions and the thermodynamic equilibrium are restored, wherein the temperature and the entropy are reduced. Thus, the temperature gradient has been spontaneously created from the motion and organizational change of the water upstream and downstream the stone.

As a model for the study of the dissipative TGM effects on water, a spontaneous temperature increase in water during daytime was used in a non-termostated room condition indoors. The study was designed in view of the effect of the temperature change and indirectly the dissipative influence of the water as an alternative to the effect of a direct motion change in view of a changed water temperature (24).

In an experiment H2 mineral water (100 ml) from the H2 water factory in Helsingborg, Sweden, was first aerated, followed by room temperature conditioning during 24 h before the experiment was initiated. It could be noted that the type of the mineral water used is not critical, and that similar results are obtained also with other variants of mineral water. The water was transferred to an open glass bottle, whereafter an electrode for temperature registration (Multilab Pilot WTW GmbH, Germany) was placed in the water. The experiment was performed during 10 consecutive days, during which control water and test water, respectively, were examined every other day during the month of August. The outdoor temperature varied between 20 and 28°C during daytime, while the indoor temperature appears from the variation in the water temperature (see Figures 1(A) and 1(B)). The temperature variation was registered by a computer every five minutes during 10 h in each experiment series with and without TGM exposure, respectively (SL matrix designned as an equilateral rhomb of the SL 1-SL5 type; Alko^{®}matrix). The X-axis in Figure 1(A) and 1(B), respectively, shows the amount of five minutes intervals. The TGM was placed directly on the surface of the glass bottle and was kept thereon during the whole experiment. Thus, the experiment was performed in daylight, but the water was not exposed to direct sunlight.

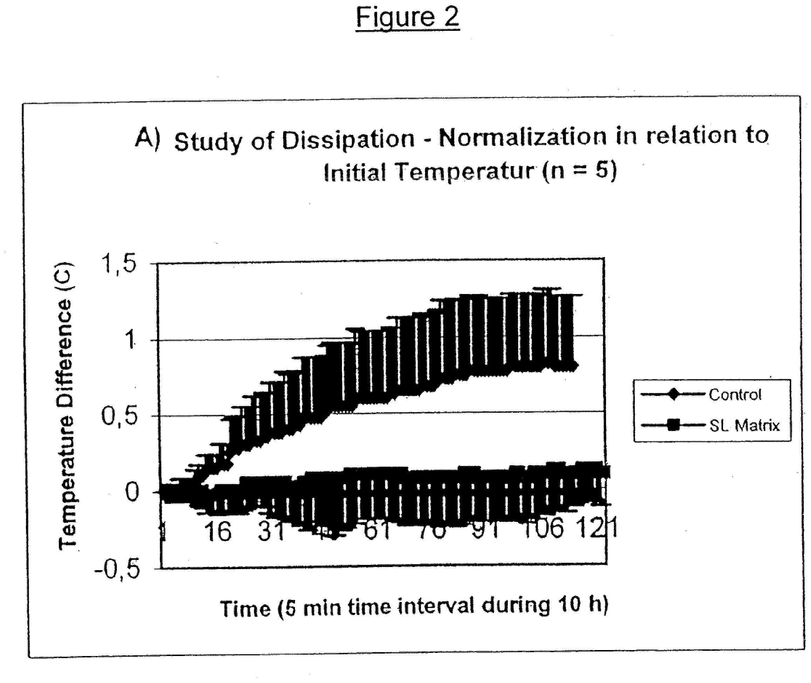

The result shows (Figure 1B) that TGM exposure creates a non-linear temperature change in mineral water with time (B), while the water temperature increases linearly (A) in control water. A normalisation (against the initial temperature) of data (Figure 2) gave a marginally increased average temperature during 10 h in TGM exposure (0.06 ± 0.04°C) (SL matrix), while the average temperature during 10 h in control water increases significantly (0.53 ± 0.25°C) (P<0.001). The normalisation gave a significant (P<0.05) temperature reduction in TGM modulation in the second half of the experiment (the last 4 hours) (Figure 2A). The result shows that TGM induces dissipative self-regulatory system changes in the water medium, which indicates spontaneous formation of synchronized water having fractal properties. It is also notable that the average of standard deviations in TGM exposure (0.23 ± 0.09) is significantly lower (P<0.001) in relation to control water (0.37 ± 0.14), which indicates higher temperature stability in synchronized water as a consequence of the cease of atomic motion and molecular mobility and migration (Figure 2A).

The temperature of a material is a measure of the motion of the atoms (25). At room temperature the velocity of the atoms is about 1500 km/h and it decreases with temperature. At the absolute zero (-273.15°C) all atomic motion has ceased. A thoroughly studied condition of material called the Bose-Einstein condensate is formed just above the absolute zero and is characterized in that single atoms lose their identity and spontaneously and suddenly couple together their motions and act like a synchronized field (13.25). The atoms follow the same wave function and assume the "laser-like" self-oscillating condition. The single particles have identical self-like or fractal properties. In the phase conversion the temperature is rapidly reduced.

The result of the experiment performed indicates that TGM induction of mineral water creates a unique condition in water at room temperature which is characterized by similar, however, limiting and slower, physical property changes, such as those taking place in the formation of Bose-Einstein condensates at the absolute zero. Typical of the process at room temperature is the slow self-regulatory formation of synchronized water, which in the study is reflected in the slow-growing temperature difference between control water and TGM exposed water. In contrast to non-synchronized control water, in which the energy uptake increases the kinetic energy and temperature of the water, the uptake of energy in TGM water is transformed to a coherent molecularly dynamic network having a high temperature stability and a fluidal co-operative structure and form. The difference between the average temperatures in each measurement thus increases with time in the 10 h experiment, which indicates a progressive self-regulatory synchronicity. It should be noted that the above-described results are also obtained if the topographic geometrical matrix would have been absent in the measurements performed, i.e. if the water would have been synchronized with the matrix in advance, as the synchronized condition of the water is stable. Thus, water in a water-containing medium could, per definition, be considered to be synchronized when, after or during TGM exposure for 10 h in daylight and at room temperature, it shows a measured average temperature increase of at most 0.10°C (0.06 ± 0.04°C), while the corresponding temperature increase in ordinary water is at least 0.5°C (0.53 ± 0.25°C) (P<0.001).

To conclude, in Example 2 it is demonstrated that TGM exposure (Aklo^{®} matrix) of mineral water induces a time-dependent non-linear temperature reduction and release of geometric entropy to the surrounding water. A dissipatively reduced water temperature feeds back to a self-regulatory synchronized condition of a thermodynamic equilibrium in the water. The condition is characterized by changes of physical properties at room temperature, which well correspond to the formation of so-called Bose-Einstein condensates, however with a specifically limited and slower temperature change. Transformed energy maintains a time-consistent, thermostable, molecularly synchronized dynamic water, i.e. a synchronized water according to the present invention.

Thus, the experiment described above allows for a person skilled in the art to distinguish between a synchronized water and a non-synchronized water (thus also between a medium containing synchronized water and a medium containing non-synchronized water).

### Example 3

Colloidal quartz (silica) may be subjected to energy induced oscillation, wherein the geometrical polyeder structure of the crystal is reorganized during formation of an energy binding polytetraeder geometry of varying size (26,27). TGM exposure was expected to release crystal bound energy from the quartz particles. Primary studies on synthetical colloidal quartz in a water suspension demonstrated that the conductivity increases linearly during the matrix exposure, while the increase is lower without matrix. The result indicates that the increased conductivity in the presence of matrix is a result of a macroscopical configurative molecular co-operation of single water molecules during formation of a synchronized stable fractal water having quartz-induced accessibility and release of crystal bound energy.

H2 mineral water was purchased for the experiment from the H2 water factory in Helsingborg, Sweden. The water was equilibrated against room air for two weeks at room temperature before analysis. The conductivity of the mineral water was determined by use of a Multilab Pilot conductivity measurement device (WTW GmbH, Germany).

Colloidal quartz (Eka Chemicals AB, Colloidal Silica Group, Bohus, Sweden), Bindzil 15/500 (BZA) and Bindzil 30/360 FG (BZB), respectively, consisting of spherical particles dispersed in a slightly basic water (BZA; 15% = 165 mg/ml, 6 nm in particle size, a density of 1.10 g/ml, a surface of 82.5 m²; BZB; 30% = 371 mg/ml, 8 nm in particle size, a density of 1.218 g/L, a surface of 133.6 m²) was diluted in distilled water in the concentrations 10, 25, 50, and 100 µg/ml. K = control water, and KM = control water treated with matrix. The preparations were left over the night before analysis. Two series of measurements were performed for initial preparations during two consecutive days.

During the experiment the mineral water was exposed to a transparent topographic geometrical matrix (TGM) in the form of an equilateral triangle (ELT). The matrix was applied directly on the storage bottle and was kept thereon during the whole experiment period.

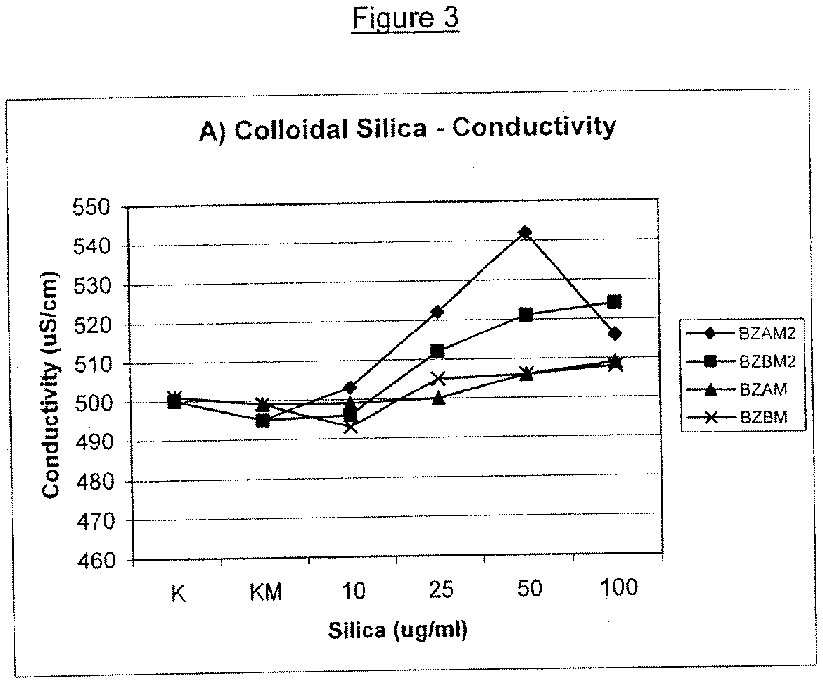

The results from the experiment are shown in Figure 3. Thus, the conductivity is reduced in the presence of matrix (K = without silica; KM = control water treated with matrix). By addition of colloidal quartz (10 µg/ml) the conductivity was slightly reduced for BZB and was unchanged for BZA. The conductivity increased for both quartz materials in concentrations above 25 µg/ml. Matrix exposure during two days (M = one day; M2 = two days) increased the conductivity linearly in relation to day 1 in particular for BZA between 10 and 50 µg/ml, so as then to be reduced at 100 µg/ml. For BZB the increase was less up to 100 µg/ml (BZA increased with 25-50 µS/cm in the range 25-50 µg/ml and BZB with 7-14 µS/cm).

Thus, water could per definition be considered to be synchronized if, in a suspension of spherical particles of colloidal quartz having a diameter of 6-8 nm each in the concentration range 25-50 µg/ml, it shows a conductivity increase of ≥ 7 µS/cm, while an ordinary water suspension with non-synchronized water shows a corresponding conductivity of <7 µS/cm.

To conclude, the conductivity increased linearly for concentrations of up to 50 µg/ml during TGM exposure, while the increase was insignificant without matrix. The result indicates that the time-dependent increased conductivity with colloidal quartz in the presence of matrix is a result of a macroscopical configurative molecular co-operation of single water molecules with formation of a synchronized stable fractal water having silica induced accessibility and release of crystal bound energy. The experiment described above shows an alternative way for a person skilled in the art to determine whether a water is synchronized or not.

### Example 4

Example 4 describes tests performed with measurements of pH and temperature oscillations for control water and synchronized water according to the present invention.

Behind the tests performed lies the discovery that there are two unique levels of physical reality, more precisely the electric level/atomic level/molecular level, that creates the physical reality in relation to the human sensory reception and cognition of electromagnetism, which also can detected and measured (45) with traditional instruments. The other aspect is represented by magnetic substance, which is present and functions in the physical vacuum (at a quantum level). These two uniquely different types of physical states seem to interpenetrate each other, but, under normal conditions, do not interact with each other (uncoupled state). In this state, the magnetic substance is undetectable with traditional measuring instruments. Via induction of a physical device a "conditioning" of a physical experimental space occurs and the two types of substance/condition can be caused to interact (coupled state). In the coupled state, traditional measurements may partially detect this vacuum level of physical reality (45). A process has been discovered for the programming of a specific intention into an electronic device. Turning such a device on in a specific space raises the electromagnetic symmetry state of the space to a significantly higher level so that coupling occurs between the two unique types of substances and tunes that space to produce a particular property measurement change. If a certain space is in the coupled state, then all the equipment therein is coupled to that state; thus all parts of the experimental system are information entangled with each other (locally and non-local parts). Conditioned spaces have several specific characteristics; (1) appearance of a vacuum level magnetic monopole, (2) temporary oscillations in the magnitude of the material measurement property, and (3) information entanglement between certain parts of an uncoupled state in an experimental system (45). The appearance of such oscillations in the material properties in a conditioned space, e.g. diumal oscillation in temperature driven by solar-induced temperature changes, measurement of pH, electric conductivity, redox potential and oxygen content in water, has been studied extensively in recent years (46). The present study aimed to investigate the influence of specifically conditioned synchronized distilled water on the oscillatory behaveour in pH measurement and its relation to diurnal variations in temperature.

For the test, synchronized distilled water (Apoteket AB, Sweden) was obtained by conditioning for 24 h in ordinary daylight with an SS matrix printed with laser on a 1 L transparent plastic bottle. The measurement of pH (WTW inoLab740, Germany) was performed with the experimental setup shielded in a Faraday's cage. The measurement procedure involved placing the pH electrode in a 30 ml polypropylene bottle filled with water. The temperature was measured in water outside the Faraday's cage close to the pH measurement equipment. Conditioned synchronized distilled water according to the present invention and distilled control water were analyzed for one week by repeated measurements collected every 10 minutes. A Fourier analysis was used for the evaluation of oscillatory frequency spectral patterns obtained from the pH and temperature profiles.

The oscillatory behaviour in pH and in temperature of distilled control water and conditioned distilled water is depicted in Figures 18A-F. The pH of control water followed an expected exponential rise with time but without identification of any significant oscillations (Figure 18A). The rise in pH was from 5.5 to 6.4. The corresponding change in temperature outside the Faraday's cage varied significantly with the typical oscillations of an expected diumal behaviour (Figure 18B). In conditioned synchronized water the rise in pH varied from 7.0 to 7.4 (Figure 18C). Previous observations of a significantly higher pH in conditioned water were confirmed in this study. The alteration in temperature was highly diumal (Figure 18D). Contrary to the control water, a significant conditioning of space in coupled state between pH and temperature oscillations was found in synchronized water (Figures 18C-E).

The thermal diumal oscillations in coupled state water are typically identified between 0.25 and 1.0 mHz (45). In conditioned synchronized water the coupled state oscillations of pH were identified in the frequency range of 4-50 µHz (Figure 18E). The highly correlated oscillations between pH and temperature as a consequence of the conditioned synchronized state have in this study typically frequencies in the low µHz range, more specifically between 4 and 15 µHz (Figure 18F), and differ significantly from the ordinary thermal diurnal oscillations in the mHz range. These coupled oscillations are also highly different and protected from alternating voltage at 60 Hz and any other source of electromagnetic influence, since the experiment was performed in an electrically grounded Faraday's cage. The absence of coupled stat oscillatory activity in the control water identifies the synchronized water as a unique coupled state medium indicating the access to magnetic monopoles, a property usually associated with a higher electromagnetic symmetry state than the normal (45). Such a higher symmetry state is also a state with higher thermodynamic free energy per unit volume, associated with a higher level of organization and reduced entropy. Also, the absence of diumal variation in temperature and pH in control water when shielded in the Faraday's cage indicates that the ordinary solar-induced unshielded oscillations are of electromagnetic origin only, while in conditioned water, besides the presence of electromagnetism, the oscillations in the µHz range probably are of solar-induced magnetic origin. Our observations may thus indicate that ordinary water in view of the temperature is under the influence of solar electromagnetism only, while the state of the synchronized water makes it highly accessible and interacting with solar-induced magnetic field lines.

Significant peaks in pH and temperature oscillations were detected at 4, 7, 12, 16, 21 and 23 µHz and are not correlated to the thermal diurnal oscillation. Interestingly, global oscillations at low frequencies have identified solar activity in the low µHz range with significant bands at 10 and 20 µHz (44).

In conclusion, the study result identifies conditioned synchronized distilled water as a unique medium with a non-thermal oscillatory-coupled state frequency profile in the range of 4-50 µHz.

### Example 5

With a view to describing the native and inducible synchronic and field-like properties of the water in relation to changes in the water chemistry, studies have been performed directly on synchronized water in view of pH, redox potential (ORP), relative hydrogen (rH), conductivity, surface tension and geometrical entropy. In Table 2 below the results obtained at a light wavelength of 634 nm and daylight are shown.

**Table 2**

| Water chemistry: characterization of conditioned H2-mineral water* | | | | | | |
|---|---|---|---|---|---|---|
| Parameter | 634 nm | | | Daylight : | | |
| 5 min | Control H2 | Control H2 634 | Matrixes | Control H2 | Control H2 DL | Matrixes |
| | Reference | Reference | Mean ± SD | Reference | Reference | Mean ± SD |
| pH | 7,083 | 7,152 | 7,180 ± 0,031 | 7,855 | 7,859 | 7,89 ± 0,012 |
| Conductivity (uS/cm) | 500 | 584 | 501 ± 1,9 | 499 | 498 | 535 ± 67,3 |
| ORP (mV) | 305 | 331 | 322 ± 4,5 | 288 | 273 | 251 ± 12.2 |
| rH | 24,3 | 25.3 | 25,1 ± 0,2 | 25.3 | 24,8 | 24.1 ± 0,4 |
| ORP (mV) Cyclic Voltammetry** | --- | --- | --- | --- | 416 | 369 |
| rH, Cyclic Voltammetry** | --- | --- | --- | --- | 31 | 29 |
| 1 week | | | | | | |
| pH | 7,612 | 7.547 | 7.010 ± 0,053 | 7,867 | T,898 | 7.890 ± 0,051 |
| Conductivity (uS/cm) | 499 | 541 | 502 ± 2,9 | 496 | 588 | 502 ± 2,5 |
| ORP (mV) | 288 | 291 | 375 ± 6,2 | 230 | 222 | 223 ± 0,6 |
| rH 2 weeks | 24,8 | 24,8 | 24,4 ± 0,2 | 23.4 | 23.2 | 23,2 ± 0,1 |
| pH | 7,644 | 7,553 | 7,637 ± 0,048 | 7,896 | 7,879 | 7,905 ± 0,042 |
| Conductivity (uS/cm) | 498 | 511 | 503 ± 3,4 | 495 | 640 | 513 ± 19,1 |
| ORP (mV) | 263 | 241 | 224 ± 6,4 | 239 | 240 | 235 ± 8,7 |
| rH | 23,7 | 23,1 | 22,7 ± 0,3 | 23,8 | 23,8 | 23.7 ± 0.4 |

| | | | | | | |
|---|---|---|---|---|---|---|
| * The characterization is related to conditioning of H2 mineral water for **five** minutes with either of the following TGM (SS, SQC, Hmatrix) and either spectral light at 634 nm of daylight. Measurements were made directly after the conditioning of mineral water and after storage in the dark at ambient temperature during 1 and 2 weeks. ** ORP was analyzed in AT mineral water by cyclic voltammetry (Department of Analyctical Chemistry. Lund University) ** AT mineral water was exposed to TGM SS during 24 h at ambient temperature. | | | | | | |

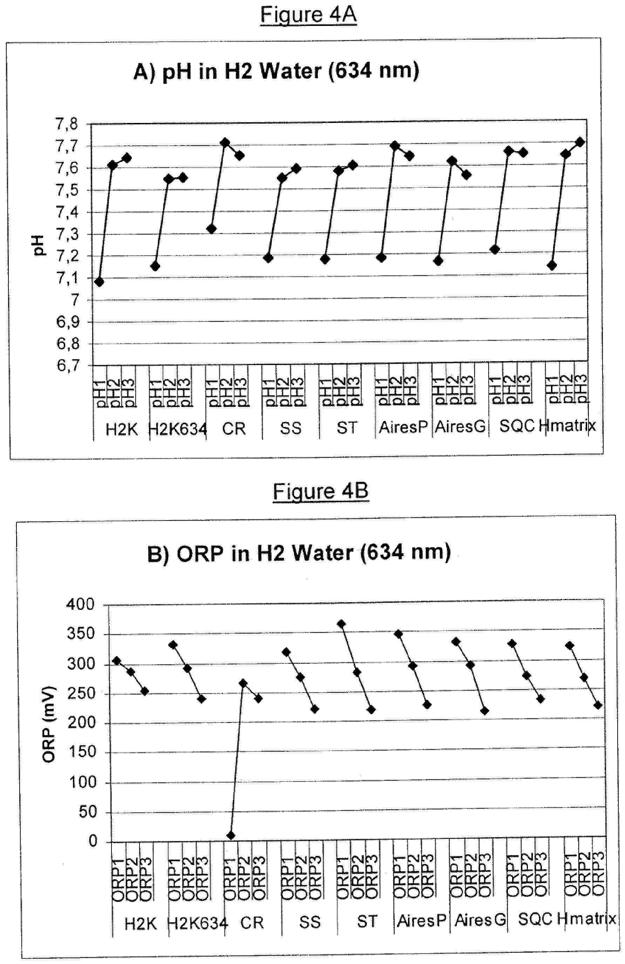

More precisely, the studies performed demonstrated that a spectral TGM modulated light induced changed chemical properties of H2 mineral water in relation to control water. Tests (not shown here) have also been performed at several different wavelengths. pH changes were observed at exposure to light at all studied wavelengths (381, 410, 476, 634, 754, 762, and 900 nm) as a function of time after exposure. The combination with matrix leads to a pH increase or reduction in relation to control water at a defined wavelength. For differentiating red light a reduced pH at 634 nm was observed (see Figure 4A) in the presence of a matrix, while the pH increased at 762 and 900 nm. The difference in pH increased with the storage time after induction with a TGM matrix. The result indicates the presence of synchronized water, and the origin and properties (effect of wavelength of spectral light) determine the actual change in electrochemical voltage potential and pH. In contrast to earlier studies, which have shown a non-linear increase in pH of an active dipole cluster (7, 12), the present studies also indicate a reduced pH (634 nm). A noted increase in pH (762 and 900 nm, respectively) indicates the presence of an active voltage potential increasing synchronized water, while a reduced pH at 634 nm indicates that a synchronized contribution reduces the electrochemical electrode voltage. The result indicates that spectral light at 634 nm influences the distribution and density of the water electrons and the dipole moment of the water molecule in a surprising way. The increased difference in pH with time indicates that the synchronizity, the local density and the stability follow self-regulating mechanisms (7,31,32).

The redox potential (ORP) constitutes a coarse measure of the reductive properties of water and the access to reductive electons. The ORP depends, inter alia, on the influence of pH variations. Relative hydrogen (rH), which is calculated from Nemst equation and which is a measure of thermodynamical electron activity, gives a better pH independent characterisation of the real reductive capacity of the water in view of the presence of low molecular antioxidative substances in a water solution. In mineral water several redox pairs having different redox potentials are present, which results in an ORP value measured comprising mixed redox potentials. At the same time redox pairs are also present in a water solution which is independent of pH and also of ORP (33). For access to real antioxidative capacity, complex biological systems/conditions with living water are needed, in which "simple" water is not accessible for reductive electrons and releases reductive capacity (33).

Hypothetically, the access to reductive electrons may increase in water depending on increased access to low density water (LDV). Low density water is characterized by more stable hydrogen bonds and lower (negative) entropy, i.e. a higher level of molecular structural organisation and higher Gibb's free energy values. The hydratization capacity then increases in a water solution, which increases the solubility of solutes in the water. An increased molecular organisation and lower entropy in water facilitate and make effective chemical reactions requiring lower activation energy than normal. Lower surface tension and increased wettability and solubility lead to consumption of less energy to wet and solve substances, which provides qualitative and quantitative advantages of functional effects in biological systems.

Systems having lower dissipative geometrical entropy, sometimes termed "entropy" for short herein, are characterized by a spontaneous higher order and organisation, wherein synchronization and self-organisation are developed (11). A unique condition is created, in which single particles are de-identified and are replaced with a condition of identical and inseparable units acting in phase in a coherent field with auto-feedback. The system shows fractal self-like properties (32). E.g., water which freezes to ice at a certain temperature shows such a condition, in which the resistance in the phase conversion between liquid and solid state suddenly is eliminated. The self-organisation is increased as the whole system is synchronized instantaneously. The system is considered as thermal dynamically "open" and shows dissipative properties emitting consumed energy (reduced entropy and temperatures) to the surroundings (31).

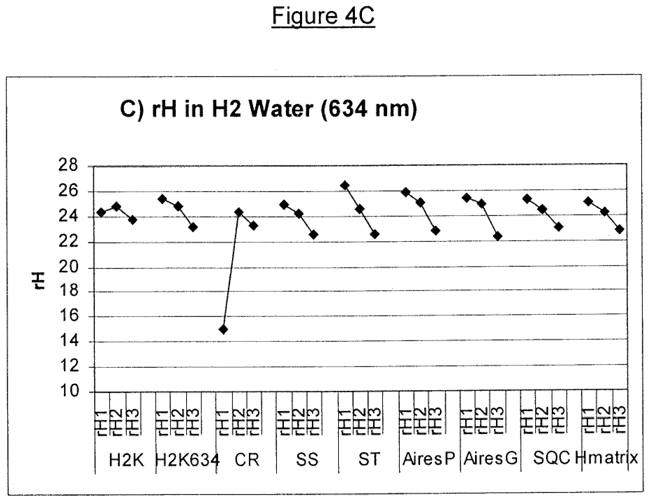

The results in our studies demonstrate that exposure to specific matrix modulated light in the red wavelength area (634 nm (Figure 4) and 762 nm) and low IR light (900 nm) reduced the ORP and the relative hydrogen, which indicates access to reductive electrons or corresponding electron energy. A reduced relative hydrogen with approximately two rH units at 634 nm, 1 rH at 900 nm, and 0.5 rH at 762 nm, i.e. within the range of 22-25 rH, indicates a powerful reductive potential and antioxidative capacity in the water. The rH scale is logarithmic, which means that a reduced rH with a factor of 1.0 corresponds to a 10-fold increase in the amount of accessible reductive electrons in the water. The breakpoint for reduction is 28 rH. Ordinary drinking water in Sweden shows rH values in the range 28-30 (32). Mineral waters studied in our examination had an rH of between 22.7 and 25.5, which shows that mineral water has a higher reductive potential in relation to drinking water. The result also shows that TGM induction further increases the reductive activity in mineral water. The increased difference in ORP and rH with time shows that these two parameters, in correspondence with changes in pH, are governed by self-regulating mechanisms.

Light at 634 nm exposed to matrix (sphere(S)) significantly also had a reduced surface tension (figure 5B), which indicates an increased wettability without addition of surface tension reducing substances. Further, an increaseed conductivity was noted in the presence of the matrix (Figure 5A). A weak conductivity increase was also noted at 762 and 900 nm two weeks after matrix exposure.

Permanent effects in view of synchronizity for pH, ORP, rH and conductivity have been observed in mineral water during storage at room temperature during one year. Increased pH indicated a self-regulative formation of stable synchronized water, while reduced ORP and rH show an increased access to reductive electrons. The reductive effect remained for at least one year after exposure.

To conclude, Example 5 showed the following:
- matrix modulated light induces water to form coherent self-stabilizing water having field-like properties,
- the self-regulation of the water adds to the development of the synchronizity with time (> 1 year after a single exposure),
- changed pH in a water solution - synchronizity leads to spontaneous self-regulative formation of stable active water,
- **increased conductivity, reduced redox potential and reduced relative hydrogen** - **facilitates and accelerates transformation of electron changes, increased antioxidative capacity,**
- **reduced surface tension** - **increases the wettability and solubility; less energy is required to wet and dissolve substances,**
- **reduced dissipative geometric entropy, increased free energy-increased solubility in water, reduces the activation energy and makes the yield of chemical reactions more effective, the presence of colloidal quartz potentiates the access to free energy.**

**With a view to examining the variability in oxidative, structure stabilized, UV protective and redox active properties regarding geometrical differences of TGM, the influence on the following biological model systems was also studied: change in enzyme activity in exposure to activity inhibiting UV light (Example 6), colloidal stability in milk (Example 7), and influence of synchronized water on the yeast Saccharomyces cerevisae (Example 8 below). The effects on the cell respiration, the redox status and the antioxidative capacity were examined.**

### Example 6

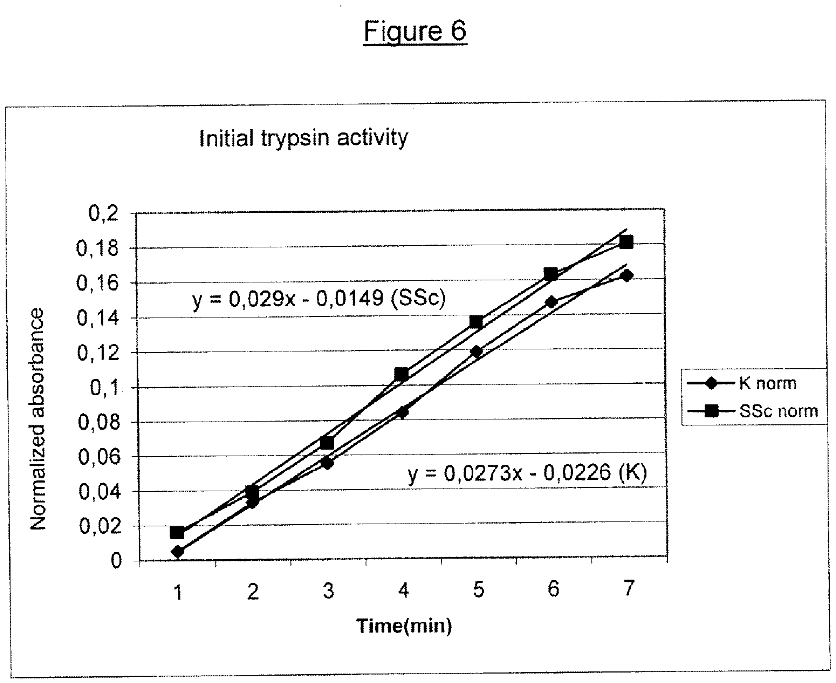

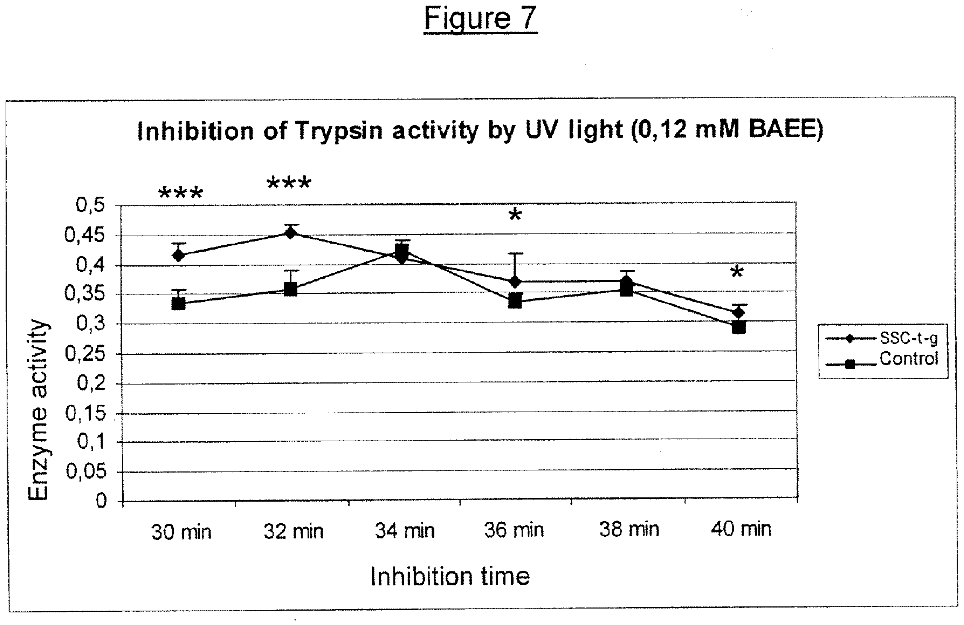

**The stimulating and UV protective properties after TGM exposure was studied in view of the enzyme activity of trypsine. Exposure of trypsine to a TGM exposed phosphate buffer stimulates the initial enzyme rate and sharpens the catalytic activity (****Figure 6****). Time-dose depending inhibition of the enzyme was observed between 30 and 40 minutes of exposure, in particular from 34 minutes. TGM exposure of the buffer during 5 minutes with spectral light at 634 nm slows down the inhibition effect of time-dose dependent UV light (****Figure 7****). Enzyme-kinetic data shows that the time dependent increaseed enzyme inhibition with an increased UV dose is significantly slowed down within the actual concentration range for the substrate. Application of Michaelis-Menten-kinetics on the inhibition process reveals reversibility of the inhibited enzyme after TGM influence. The Michaelis constant** (K_{M}), **calculateed from a non-competitive inhibition reaction, is reduced, as well as the maximum enzyme rate (Vₘₐₓ). The result indicates that TGM induces a macro-molecularly synchronized water having potential to stabilize the tertiary struc**ture of the enzyme and to increase the affinity of the active center for the substrate, independent of simultaneous influence of protein denaturating ionizing UV light. It should particularly be noted that the catalytic properties of the enzyme are increased in spite of the UV exposure. Thus, the synchronized water according to the present invention is useful as a replacement of chemical UV protective substances in skin creams, or alternatively as a replacement for UV light for preservation of foods.

### Example 7

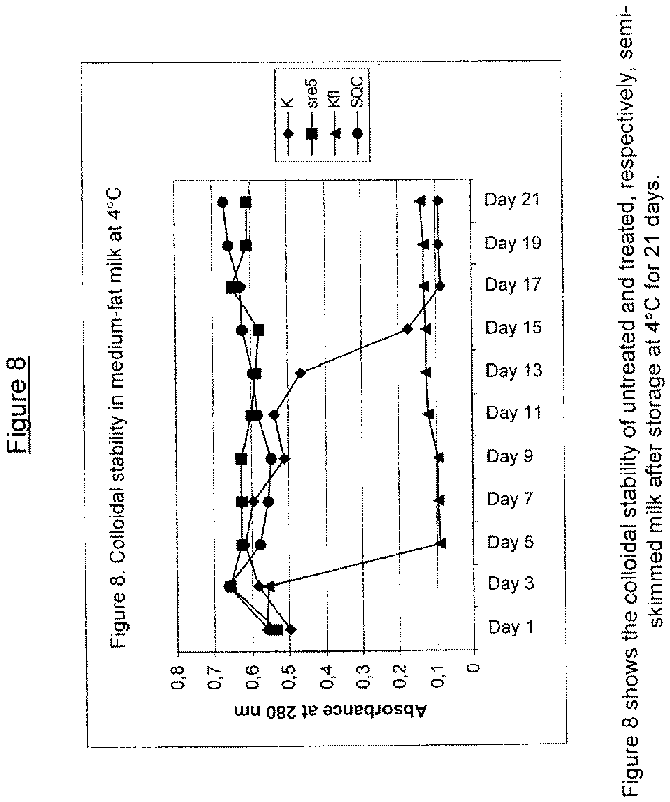

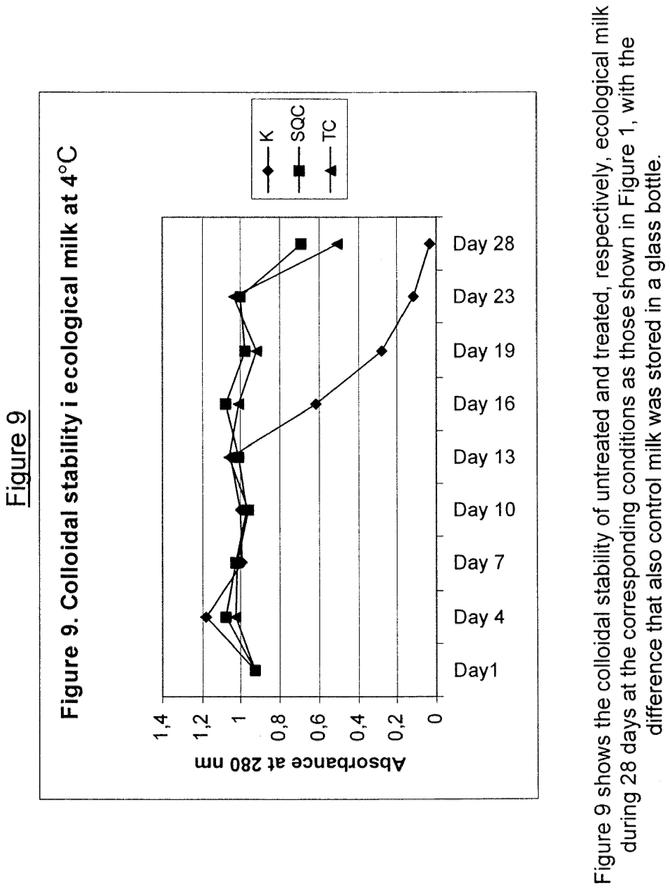

During studies of semi-skimmed milk (Figure 8) and ecological milk (Figure 9), respectively, after continuous exposure to TGM and SRE matrix, respectively, for up to 30 days at 4°C, increased colloidal properties, reduced oxidation and chemical and biological stability were developed for several of the matrixes during the test. The recommended final day of consumption during the test shown in Figure 8 was day 6. A colloidal stability of milk stored in its original package began to degrade on day 7. Milk stored in transparent glass bottles was dramatically impaired after day 3. Milk stored in a glass bottle was fully oxidized and non-drinkable on day 5 and milk in its original package on day 15. Milk exposed to Aklo^{®}matrix (SQC) and SRE antenna, respectively, showed increasing colloidal properties after day 15 and these remained also after day 21. Both SQC and SRE exposed milk were unaffected, ocularly and as to smell unaffected by the oxidation when the test was finalized on day 21. The recommended final day of consumption was day 5 in the test shown in Figure 9. A remarkable difference between the results in Figure 8 and Figure 9 is that ecological control milk showed an increased stability in relation to ordinary medium fat milk during storage in a transparent glass bottle. The exposure of milk indicates that the matrixes (SQC, TC) show a similar property profile in view of induction of increased colloidal stability.

The result indicates that the milk water may slow down the oxygen- and photodriven oxidation of both milk protein and unsaturated fat in the milk. The milk was subjected to continuous exposure to aerobic micro-organisms. Also in this regard the microbial oxidative influence was probably slowed down. The stabilizing quality-increasing consumption properties observed may (probably) generally be transferred to liquid based foods, beverages and pharmaceutical products, and may in such cases replace, alternatively complement, conventional preservation agents. Thus, the test shows that synchronic properties may be transferred to the biological water medium of milk and increase the qualitative durability of milk and add a functional health added value. Thus, the synchronized water according to the present invention may be used as a preservation agent or as a complement to preservation agents in foods, such as dairy products and beverages, and to add a health added value, e.g. in the form of a so-called functional water.

Functional beverages based on the present invention may be used to restrict the epidemical development of lifestyle related diseases, such as obesity, hypertension, and type 1 and 2 diabetes, and by reinforcement of the physiological health stimulating self-regulation and/or by offering a complement to control diet related ill-health.

### Example 8

During studies on yeast the accessibility to synchronized water, induced via TGM (sphereS), contributed to stimulation of mitochondrial activity, which resulted in an increased reductive and antioxidative capacity of yeast cells (see Table 3 below). An increased oxygen consumption indicated access to dissipative electron mediated free energy, in addition to addition of electrons via ordinary oxidation of glucose. The water in the cell culture showed synergism and co-operativity via a lower geometrical entropy. The reducetion of entropy showed that yeast cells are able to transform free energy directly from synchronized water and to utilize the energy for oxidative processes in the cell. During nutrient-poor conditions synchronized water induced a shift from oxidative to reductive metabolism, a reduced oxygen consumption and a lack of extracellular production of antioxidative substances. The result confirmed that antioxidation in yeast cells is oxidatively regulated and ATP dependent. Thus, synchronized water showed adaptogenic redox activating properties. Aerobical conditions and nutrient access stimulated mitochondrial activity and extracellular antioxidation, while starvation induced reductive metabolism and inhibited transmembral distribution of antioxidants.

From a mechanistic viewpoint, the observed advantageous effect of TGM on cell respiration and antioxidation of yeast cells in nutrient access in relation to lack of antioxidation in starvation may probably exclude intracellular access to reductive electrons directly in water. One alternative in the present knowledge situation is that the synchronized water's developed self-organisation and synergism makes energy accessible in mitochondrial environment from e.g. delocalized electrons or corresponding energy (free energy) from synchronized water to electron-driven processes in the mitochondrie. Synergism reduces the surface tension, makes the accessibility to dissolved substances more effective, stabilizes the configuration of enzymes, stimulates intra- and extracellular transport, as well as catalytic enzymatic activity, thereby contributing to a more effective cell respiration. Yeast cells exposed to synchronized water may be used for the production of an antioxidative cocktail having high stability against oxidation, which may be used as a functional water or add a health added value to established beverages, e.g. juices and "health beverages".

**Table 3**

| Yeast cells inoculated in synchronized water * | | | | | | | |
|---|---|---|---|---|---|---|---|
| Culture media** | Control | Sphere(S)634 | SRE | CR | P* | P^{b} | P^{c} |
| pH | 5.688 ± 0,152 | 5,872 ± 0,151 | 6,090 ± 0,023 | 8,035 ± 0,426 | <0,05 | <0,01 | <0,01 |
| ORP (mV) | 289 ± 5,5 | 276 ± 8,1 | 268 ± 9,9 | 212 ± 24 | <0,05 | <0,05 | <0,01 |
| rH | 21 ± 0,1 | 20,9 ± 0,3 | 21.1 ± 0,3 | 23,1 ± 0.7 | NS | NS | <0,05 |
| Oxygen (mg/l) | 4,97 ± 0,24 | 5,08 ± 0,15 . | 5,08 ± 0,17 | 5.07 ± 0,14 | NS | NS | NS |
| Geometric Entropy | 2,36 ± 0.19 | 1,66 ± 0,22 | 1,48 ± 0.25 | 1,75 ± 0,28 | <0,001 | <0.001 | <0,001 |

| Yeast, Nutrition** | Control | Sphere(S)634 | SRE | CR | P^{a} | P^{b} | P^{c} |
|---|---|---|---|---|---|---|---|
| pH | 3,122 ± 0,188 | 3,151 ± 0,141 | 3.124 ± 0,156 | 3,261 ± 0,196 | NS | NS | <0.05 |
| ORP (mV) | 389 ± 30 | 348 t 8,2 | 338 ± 5,6 | 319 t 7.5 | <0,05 | <0.05 | <0,01 |
| rH | 19,2 ± 1.2 | 17,8 ± 0,4 | 17,5 ± 0,4 | 17,2 t 0.5 | <0.05 | <0,05 | <0,05 |
| Oxygen (mg/l) | 3,67 ± 0,27 | 3,05 ± 0,40 | 2.85 ± 0,59 | 2,88 ± 0,35 | <0,05 | <0,05 | <0.05 |
| NaOCI reduction (umol/3,5 ml) | 11,9 ± 1,4 | 14,7 ± 1,6 | 14,1 ± 1,5 | 13,2 ± 1,7 | <0,05 | <0,01 | <0,05 |
| Geometric Entropy | 2,13 t 0.22 | 1,92 ± 0,21 | 1,72 ± 0.25 | 1,91 ± 0,36 | <0,05 | <0,01 | NS |

| Yeast, Starvation** | Control | Sphere(S)634 | SRE | CR | P^{a} | P^{b} | P^{c} |
|---|---|---|---|---|---|---|---|
| pH | 4,601 ± 0,355 | 4.634 ± 0,362 | 4,698 ± 0,383 | 5,799 ± 0,586 | NS | NS | <0.01 |
| ORP (mV) | 283 ± 27 | 273 ± 31,8 | 270 ± 31,5 | 221 ± 25,8 | NS | NS | <0,01 |
| rH | 18,6 ± 0,6 | 18,4 t 0,4 | 18,4 t 0,3 | 19,0 ± 0.4 | NS | NS | NS |
| Oxygen (mg/l) | 3,56 ± 0,13 | 4,16 ± 0,43 | 4,19 ± 0,54 | 3,95 ± 0,22 | <0,05 | NS | <0,05 |
| NaOCI reduction (umol/3,5 ml) | - | - | - | - | - | - | - |
| Geometric Entropy | 1,97 ± 0,37 | 1,94 ± 0,30 | 1,90 ± 0,24 | 1,88 ± 0,33 | NS | NS | NS |

| | | | | | | | |
|---|---|---|---|---|---|---|---|
| * Statistics related to a comparison between study groups (vertical relation) are not shown in Table 3. All parameter (pH, ORP, rH, Oxygen) were then compared between groups different significantly (P<0.01-0.001). P^{a} t-test between groups; Sphere(S)634 vs control. P^{b} t-test between groups; SRE vs control. P^{c} t-test between groups; CR vs control. ** The values of measurement of pH, ORP, rH, oxygen and geometric entropy is a mean value from three independent measurements.The reduction of NaOCl was estimated as the mean value of three repetitive measurements at three different occasions. | | | | | | | |

To conclude, the studies on yeast cells, inoculated in synchronized water (photobiomodulation of TGM exposed light at 634 nm), show that stimulated adaptive oxidative metabolism and intra- and extracellular, respectively, redox activity mechanistically involve spontaneous self-regulation and dispersive property changes in the synchronized water.

### Example 9

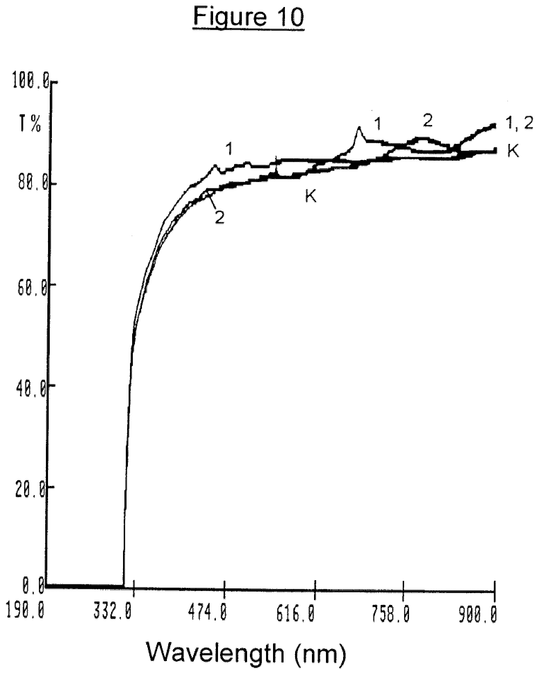

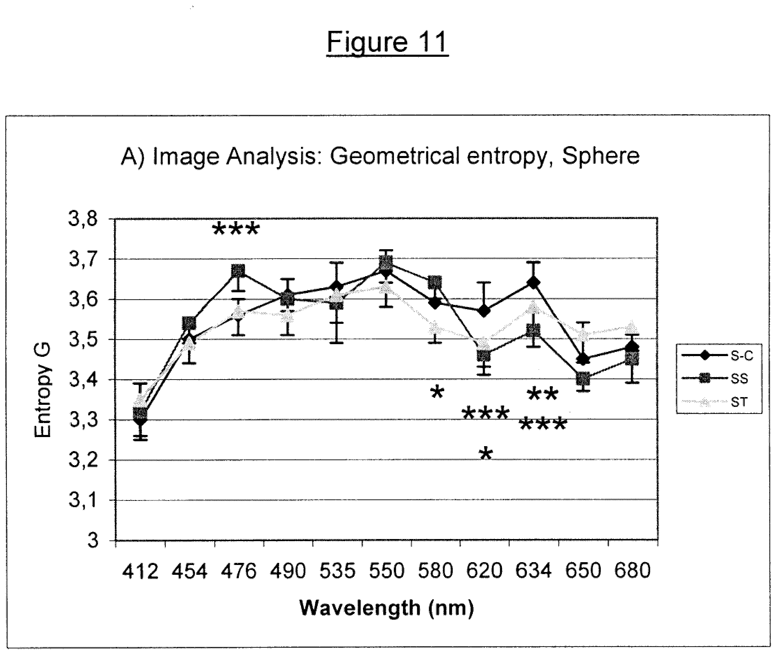

During induction of changed electromagnetic properties in spectral transmitted visible light, distinguishing effects were noted in the form of increased or reduced transmission of light in the visible and lower IR region (330-900 nm)(Figure 10)(Table 4). Generally, a transmission reduction for wavelengths below approximately 630 nm was noted, while the light was not affected between 700 and 800 nm, or a certain transmission increase at wavelengths in the range 800-900 nm. A reduction in light transmission indicates an electromagnetic energy change in the light between 330 and 630 nm without influence at wavelengths between 700 and 900 nm in the presence of the matrix. The variability in transmission cannot be explained by the effect that visible light is absorbed/reflected by the presence of the geometry printed on the matrix surface, as matrixes having the highest covered area on the matrix showed a higher light transmission and that transmitted visible red and low IR light were not affected by the presence of the covered area. Further, an increase of the transmission in turquoise-blue colouring of the matrix was noted in relation to black colouring at wavelengths below 630 nm. The reason why the transmission is reduced at wavelengths between 330 and 630 nm is that the physical properties of lights are changed due to interference via cymatic geometry and spectral light in combination. The studies showed at the same time that visible light, mainly in the red wavelength range, reduced the geometrical entropy of matrix transmitted light (Figure 11). The result shows that the electromagnetic spatial and organisatory properties of light can be influenced. Of particular interest is the reduction in geometrical entropy for red light, which suggests that chaotic photons emitted from an ordinary light bulb are reorganized by the matrix with an increased presence of co-ordinated oscillations, creating a light flow with laser-like properties, wherein the light acts as a more coherent self-oscillating field (6). The observation may also explain the increased intensity of modulated light in the wavelength range 800-900 nm (6).

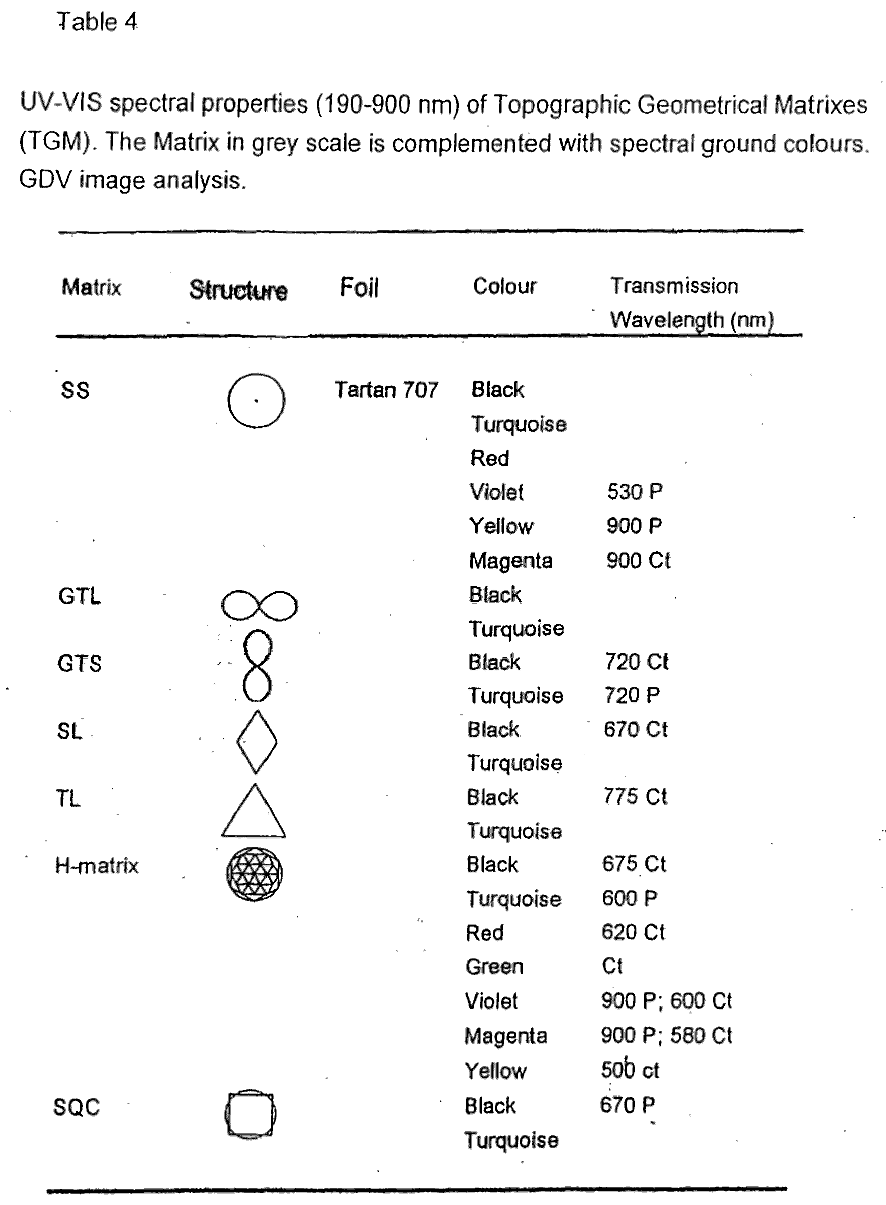

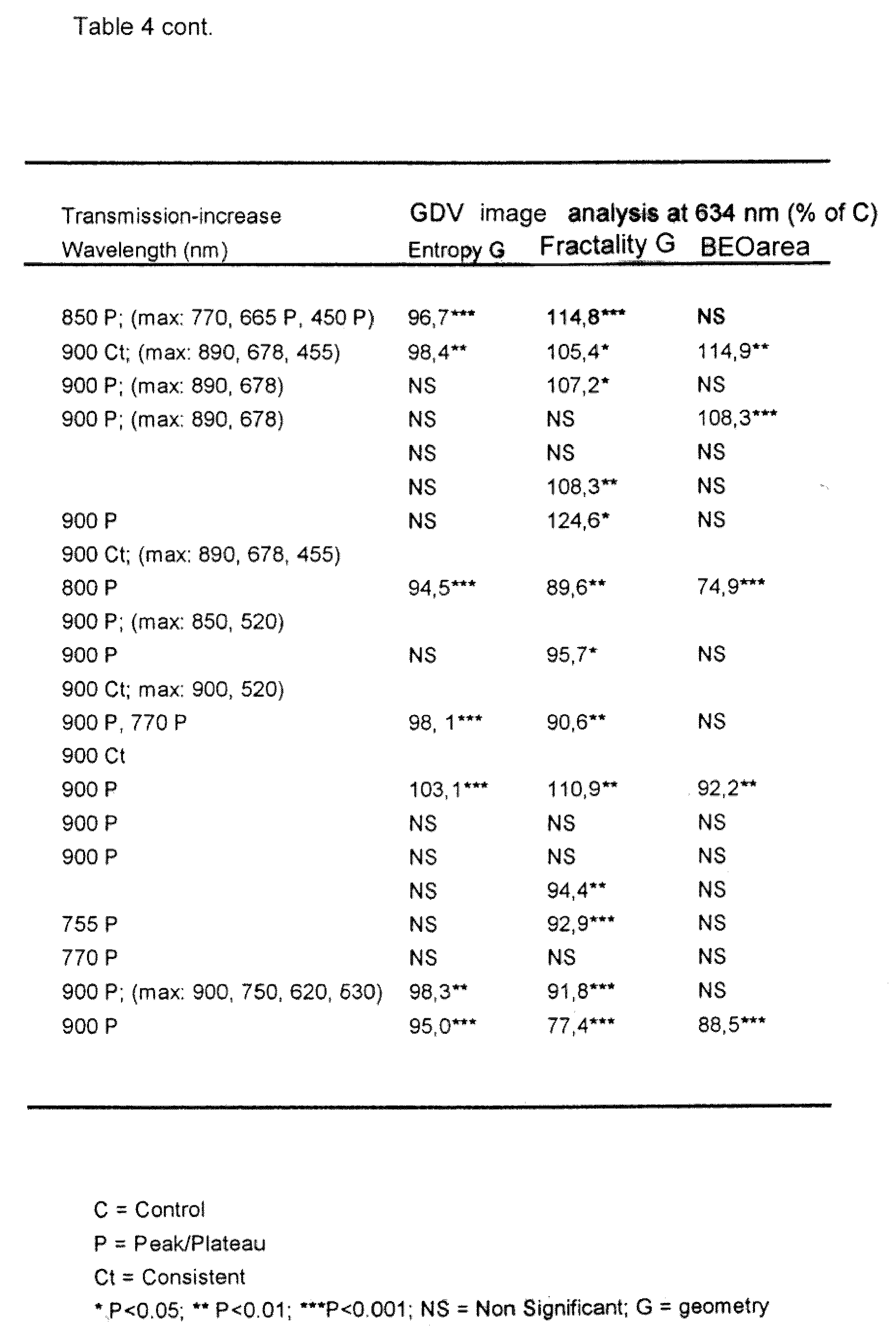

Thus, the following was shown:
- topographic geometrical matrixes (TGM) modulate physical properties of ordinary visible light during formation of light having coherent laser-like properties,
- the light shows an increased co-operation and organisation of single wave components of both electrical and magnetic nature,
- TGM induces self-stabilizing electromagnetic field having fractal structure and organisation.

### Example 10

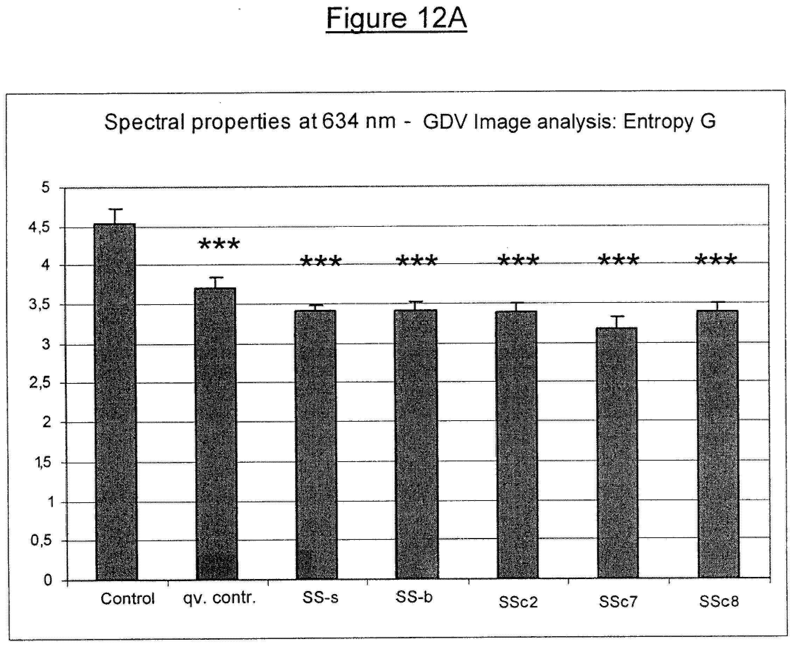

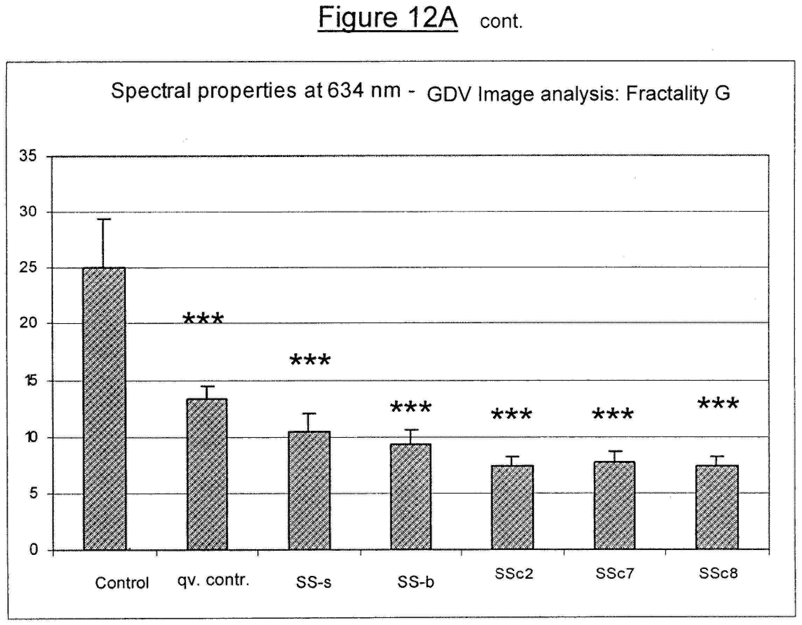

Dispersive properties of topographic matrixes (TGM) (based on the basic geometry of the circle and the sphere) were studied in view of spectral light (from a photometer) at 634 nm after printing on glass or plastic foils (Figure 12A). The topographical geometry was studied according to the following; mono-, di-, and triple circle, respectively (circle, dicircle, tricircle), closed and open sphere (SS, SSc), laying and standing giving sign (GTL, GTS), rhombogram (SL), squared circle (SQC), equilateral triangle (TiC), circular metal rings made of copper and brass, respectively (13 mm in diameter, 1 × 5 mm) and an SRE antenna (7 re-coupled circular rings on a spool). The matrixes were imprinted on an optical cover glass (Göteborgs Termometer-fabrik) and optical quartz glass (18 mm x 1 mm, Schott Lithotec, Germany). The imprint was performed by ink, letter press and gold-leaf, respectively, on cover glass and quartz glass. The impact of the line width of the outer circle and the diameter of the inner circle, respectively, as well as the line width and the imprint of the matrix on the front side in relation to incident light, was examined on a quartz matrix.

The results were evaluated by use of optical imaging via a Kirlionic GDV processor (29b) and are present in Figure 12A. Studies on optical cover glasses imprinted with ink showed a significant (P<0.001) reduced entropy for TGM according to the following: circle, SS, SSc, GTS and GTL. The reduction in entropy increased for SS and SSc with gold-leaf and letter press imprint.

The imprint of TGM on optical quartz glass (ink) gave a significantly reduced entropy (P<0.001) for a circle, SS, SSc, GTS, SL (0.01), SQC and a tricircle. The result also suggests that the entropy reduction becomes more pronounced with the imprint against light. The quartz passage probably also increases the electromagnetic organisation and co-operation of the light. Imprint with gold-leaf further reduced the entropy of the studied matrix (SS).

The line width of the outer circle was 0.1 mm or 0.5 mm for three of the matrixes (circle, SS, SL). The result demonstrates that the two first-mentioned matrixes show a significantly reduced entropy (P<0.001) at a line width of 0.5 mm, in contrast to 0.1 mm, for which the effect corresponded to the control value.

The diameter of the inner open circle and the line width of the matrix in view of entropy reduction has the most pronounced effect with quartz glass in the following ranking; SS7 (outer circle: 13 mm x 0.5 mm, inner circle: 3 mm × 0.1 mm) (diagram 1) > SSc2 (inner circle: 2 mm x 0.1 mm) = SS8 (inner circle: 3 mm × 0.35 mm)(Figure 12A) >SSc1 > SS > SSc3 > dSSc > SSc6 (Figure 12A). The matrixes SSc4 and TiC, respectively, gave a significant (P<0.001) entropy increase. To conclude, the result from the studies of cover glass and quartz glass, respectively, shows that the entropy reduction throughout was most powerful for SSc(3), and thereafter for SSc(2), SS and GTS. For SS it was noted that the diameters studied on the inner ring gave a similar entropy.

The metal matrixes consisting of copper and brass also show a powerful entropy reduction (P<0.001), wherein the copper matrix is most effective. Exposure to an SRE antenna also leads to a significant entropy reduction (P<0.001) in the presence of quartz glass outside the ray path.

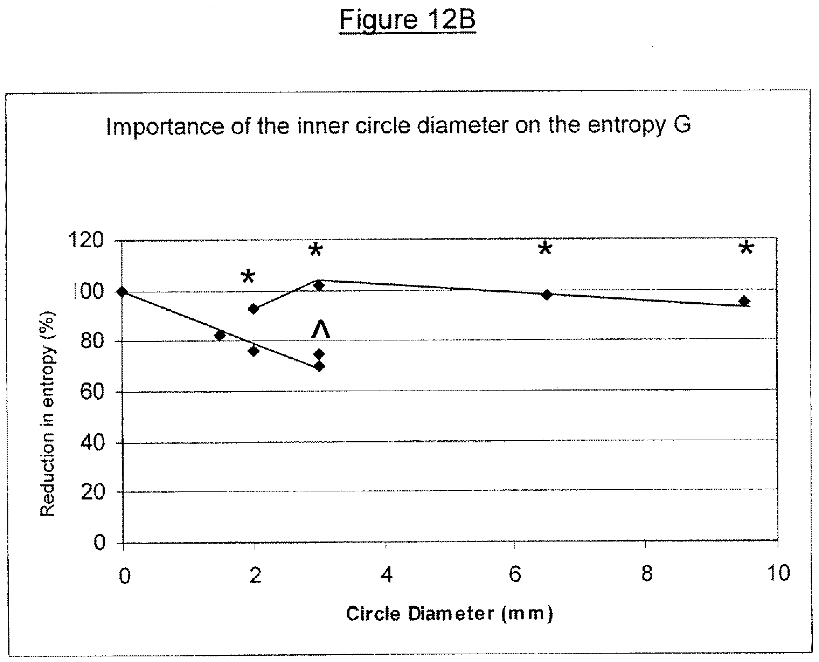

In Figure 12B the relationship between the reduction in entropy G and the inner circle diameter of the SSc matrix, as well the importance of the line width of the circle is shown. For the line width of 0.1 mm the entropy G is linearly reduced up to a circle diameter of 3 mm. An increase of the line width to 0.035 mm and 0.5 mm, respectively, leads to an increased entropy at the corresponding circle diameter (2 mm and 3 mm, respectively). The result shows that the entropy is dependent on the proportions of the inner circle and follows a linear relationship between reduced line width and reduced entropy G. With a view to achieving a low entropy G, an inner circle diameter of at least 3 mm and a circle line width of at most 0.1 mm are required.

To conclude, it could be noted that TGM imprint with an SSc matrix (outer circle: 13 mm × 0.5 mm, inner circle: 3 mm × 0.1 mm) on an optical cover glass and a quartz glass, respectively, leads to a powerful entropy reduction of spectral light of 634 nm. The effect is similar for an imprint with a letter press and gold-leaf, respectively. Also, a circular metal matrix made of copper (13 mm in diameter x 1 mm x 5 mm) gave a corresponding entropy reduction. With a view to achieving a low entropy G with imprinted matrixes, an outer line width of 0.5 mm is required, wherein the inner circle diameter is at least 3 mm and the line width is at most 0.1 mm.

### Example 11

In one experiment the spatial physical properties of light with a wavelength of 634 nm were measured with matrixes of different kinds imprinted on quartz glass or boron silicate glass. The entropy G, fractality G and BEO area were measured. The results are shown in Table 5 below.

**Table 5**

| Physical (spatial) properties of light. Characterisation of spectral light at 634 nm. | | | | |
|---|---|---|---|---|
| Parameter | Quartz glass at 634 nm | | Borosilicate glass at 634 nm | |
| | Control | Matrixes | Control | Matrixes |
| | Mean ± SD | Mean ± SD | Mean ± SD | Mean ± SD |
| Entropy G | 3,55 ± 0,26 | 3,31 ± 0,16*** | 3,57 ± 0,28 | 3,32 ± 0,24*** |
| Fractality G | 9,68 ± 4,68 | 6,04 ± 2,44*** | 7,03 ± 2,37 | 5,86 ± 1,76*** |
| BEOarea** | 15625 ± 1537 | 16307 ± 1948*** | 17671 ± 1490 | 18408 ± 3308*** |

| | | | | |
|---|---|---|---|---|
| * The characterization is related to evaluation of spatial changes in qualities of spectral light at 634 nm with either of the following TGM (SS, SSc, GTS). Measurements were made directly on light passing the TGM matix imprinted on either quartz or borosilicate glass. Physical parameters were analyzed by image analysis. ** Unit; pixels. *** P < 0.001 ; n = 110. | | | | |

The experiment showed that conditioned light shows a substantial reduction of entropy G and fractality G with TGM imprinted on both of the glass types. The BEO area was similarly substantially increased for both study groups.

A reduction in entropy indicates a higher condition of spatial organization of electromagnetical waves in conditioned light. The reduction in fractality confirms a condition of increased self-similarity of the interacting electromagnetic waves. The BEO area represents an increase of the light intensity, i.e. a greater amount of photons was detected with conditioned light.

### Example 12

A spectrophotometer based study of the effects of light exposure with and without a topographic matrix on CaCO₃ precipitation was performed. Through the development of Higashitani et al (41), Kney and Parsons (2006) have increased the reproducibility of the laboratory trials. Kney and Parsons have focused on the conditioning of all the glassware and they have also made some changes in the assay. During the experiment performed here, the inventors behind the present invention have utilized Kney and Parsons method with a view to examining if there is a similarity between deionised water exposed to a magnetic field (16 min at 5500 x G) and deionised water exposed to the matrix according to the present invention (an SS matrix), i.e. synchronized water according to the present invention.

During the experiment solutions of 0.011 M Na₂CO₃ and 0.008 M CaCl₂ were first prepared from deionised water (Aqua purificata eur, Swedish Apoteket) and salts from Scharlau (Na₂CO₃) and SERVA (CaCl₂). The cuvettes used in the spectrophotometer tests were conditioned according to a schedule made by Kney and Parsons (42). During the conditioning of the cuvettes, the following steps were performed:
1. The inside of the cuvette is swabbed with a Q-tip soaked in 5% HNO₃ followed by 3x rinse with deionised water (DI water).
2. The inside of the cuvette is swabbed with a Q-tip soaked in 0.5% NaOH followed by 3x rinse with DI water.
3. The inside of the cuvette is swabbed with a Q-tip soaked in 0.011 M Na₂CO₃ followed by 3x rinse with DI water.
4. Fill the cuvette with 0.011 M Na₂CO₃ and let soak for 1 minute, followed by 3x rinse with DI water.

The test procedure was performed as follows:
Preparation of 200 µl seed in cuvette 1
   1. Mix 1.5 ml 0.011 M Na₂CO₃ with 1.5 ml 0.008 M CaCl₂, draw the combined solution in and out of the pipette two or three times and discard the pipette tip once completed.
   2. The seed cuvette is immediately exposed to an SS matrix and daylight for 5 minutes. The control is treated in the same way, but without the SS matrix exposure. Remove 200 µl of the seed suspension after exposure and add to the test cuvette.
Preparation of test cuvette 2
   1. Mix 1.5 ml 0.011 M Na₂CO₃ with 1.5 ml 0.008 M CaCl₂, 25 µl 0.5% NaOH and 200 µl seed suspension.
   2. Mix the combined solutions in and out of the pipette two or three times. Discard the pipette tip once completed.
   3. The test cuvette is immediately placed into a spectrophotometer chamber and the test is initiated.

Absorbance measurements were made using a UV/Vis spectrophotometer (PG instruments T80+). Measurements were taken at 5 s intervals over a 30 min period at 350 nm. The peak absorbance was observed when the maximum number and size of particles were reached, and after that a decrease due to sedimentation and/or crystallization was observed.

The result of the experiments performed shows that the effects of using a seed suspension from a mix of 0.011 M Na₂CO₃ and 0.008 M CaCl₂, exposed to an SS matrix in daylight, are similar to the effects found by Kney and Parsons when exposing the mixture to a magnetic field (42). The SS matrix exposed mixture had a significantly (P<0.05-0.01) higher precipitation velocity (between 740 and 1550 s after the start of the precipitation) than the control. This observation indicates that the combination of a topographic matrix and light induces a change in the magnetic component of ordinary daylight, which might influence the dielectric properties of the conditioned water.

This appears from the results shown in Figure 17.

The increased precipitation velocity is probably due to a difference in both the size and the form of the crystals. According to Kney and Parsons (42) the pH has a significant effect on the results of the tests. They found that small changes in pH produce varying sedimentation and precipitation conditions for the crystals produced. When the pH was measured in this study, it was slightly higher with the matrix than without (pH_{control} = 11.133 and pHₘₐₜᵣᵢₓ = 11.202; P<0.001 mean value out of 3 measurements (N = 3), WTW inoLab740). This could be a part of the explanation why the kinetics differs.

The higher pH refers to a reduction of the proton content in the conditioned water and reduces the dissociation of the water molecules to proton and hydroxyl ions. From an earlier study (43) the dielectric constant of the conditioned water was found to be substantially increased. As this change in the dielectric properties of the water refers to the hydrogen bond character, i.e. both the strength and the extent of hydrogen bonding was increased, it might also be a pronounced effect on the weak van der Waals bonds between and among different structural units in water. The change in the dielectric constant exerts a stabilizing effect, probably due to influence of the magnetic dipole of water, which makes the movement of the molecular water dipole difficult and restricts the ability of the water molecule to oscillate at the matrix induced "specific" frequency. The observed increase of the pH during the use of the SS matrix has also been noted from other experiments with conditioned synchronized water in the present application text.

### Example 13

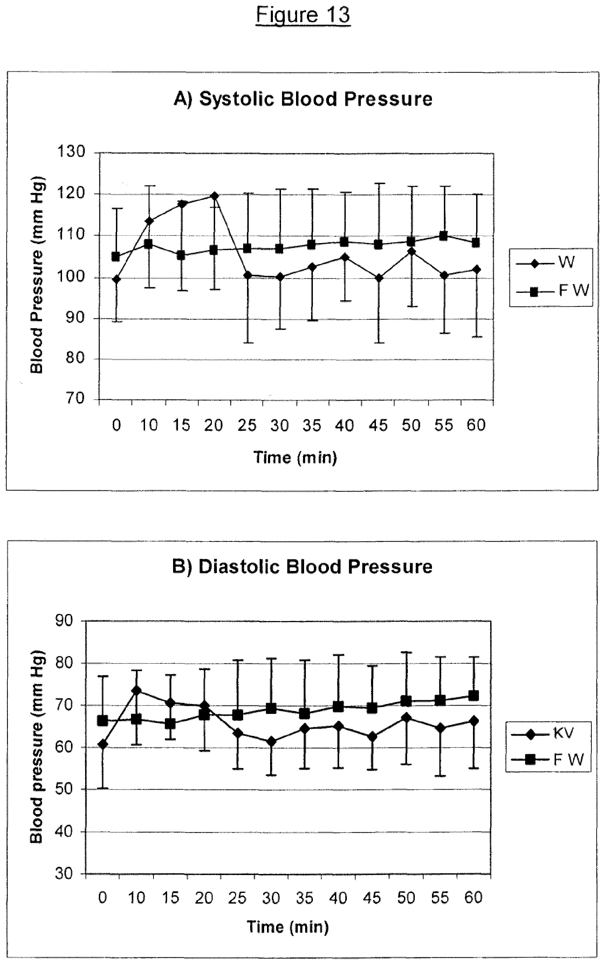

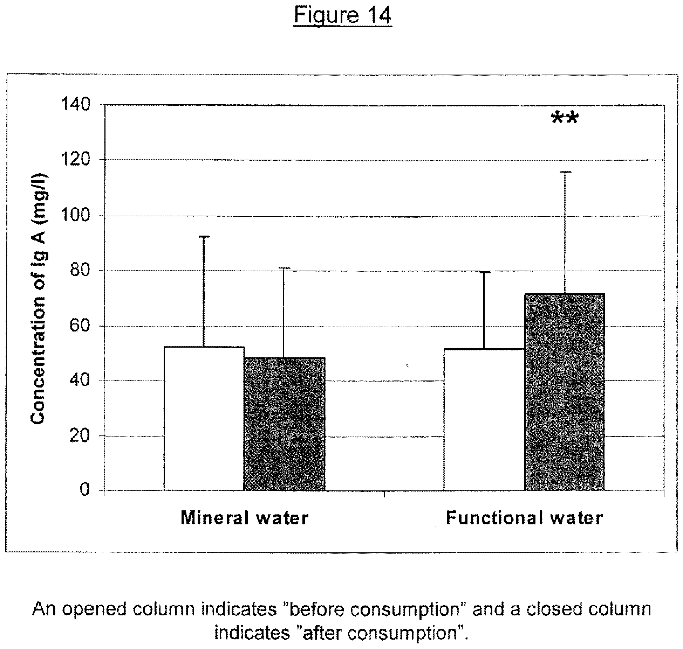

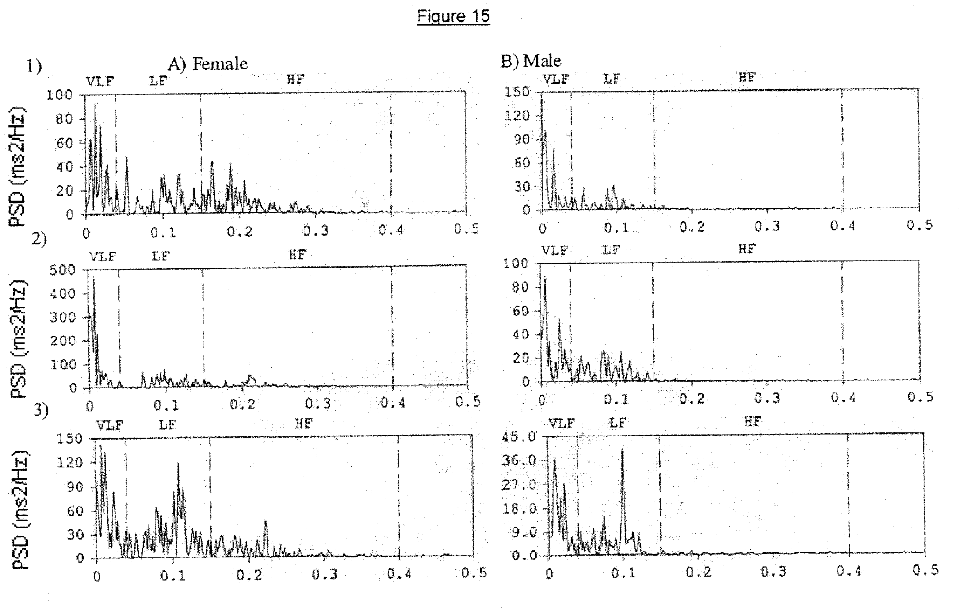

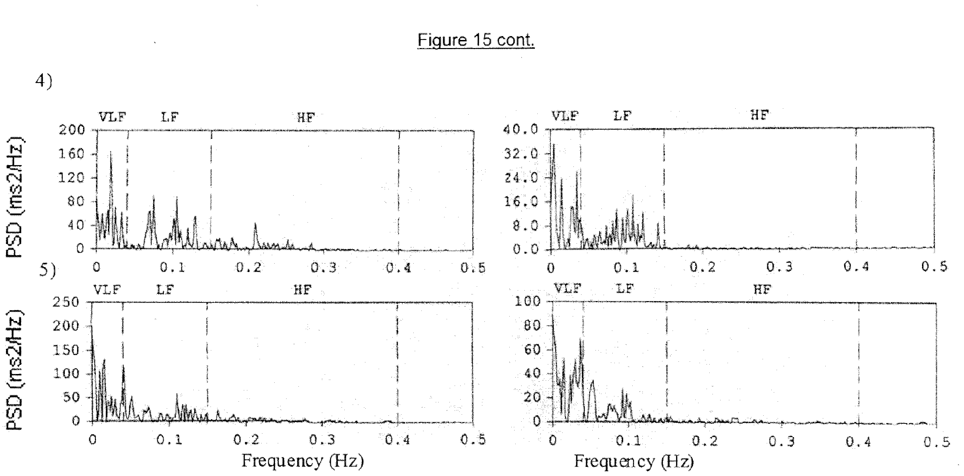

Studies on human beings have shown that consumption of synchronized water as a beverage (Figure 13 and 14, respectively) and during exposure in gas phase (Table 3 and Figure 15) stimulates parasympathetic activity in the central nervous system with a positive effect on humoral immunity in saliva via increased secretory access (46%) of immunoglobulin A (IgA). The result also indicates normalisation of the blood pressure in relation to a known blood pressure increase with ordinary water. In both studies it was noted that synchronized water according to the present invention induces and conditions a physiological condition which is characterized by an increased autonomously regulated parasympathetic activity, associated with a reduced heart rate, increased heart rate variability (HRV), vagal activity and sympatovagal balance. A spectral frequency analysis identified a reinforced band in the frequency region around 0.1 Hz (5), which indicates that the exposure to synchronized water leads to an increased systemic autonomous stability. The effect on humoral immunity (Figure 14) confirms that the access of IgA is autonomously controlled and that the concentration increase in saliva is para-sympathetically regulated and stimulated by synchronized water in gas phase.

Thus, Figures 13A and 13B show that consumption of synchronized water normalizes induced blood pressure increase after consumption of ordinary water. Further, Figure 14 shows that synchronized water stimulates secretory humoral immunity in saliva.

The reduction in heart rate further indicates that synchronized water has an effect on mitochondrial cell respiration and probably makes the energy yield and oxidative production of chemical energy (ATP) more effective by correspondingly about 3% in relation to the reduction of the heart rate. A reduced heart rate in the absence of simultaneous change in respiratory sinus-arrythmia and oxygen consumption could be interpreted to mean that synchronized water in gas phase adds dynamic synergism, self-organisation and a dissipative functionality to the body water. A more effective metabolism which has been observed in induction of synchronized water to yeast cells (see Example 8) could thus be indicated and directly be transferred to water in a liquid and gas phase, respectively, and be transferred to the body water of a human being. Thus, synchronized water could add to a progressive functional adaptivity in autonomously regulated biochemical and physiological processes of the human being and stimulates adaptive physiological homeostasis. Further, it was observed that an autonomously mediated cardiophysiological load during exposure to an electromagnetic field from a computer screen was neutralized by synchronized water in gas phase, which appears from Table 6 below.

During the experiment, the result of which is shown in Table 6, 50 healthy volunteers were placed in front of a computer screen, and the ECG was measured for 10 minutes in five sequential tests. Initially the computer was closed. Thereafter, the computer was started during the following four measurements. During tests 3 and 4, respectively, the relative order between two different begonia plants cultured with control water and synchronized water (active), respectively, was randomized, and they were placed on the right side of the computer screen. The heart rate, the heart rate variability (HRV) and the so-called Power density parameters (PSD) were analyzed according to medical criteria. The experiment showed that the heart rate was reduced and that the HRV increased in the presence of and finally (test 5) also in the absence of active begonia. During the same conditions the PSD showed an increased total effect and increased intensity at low and high frequencies. With the computer switched on an increased sympathetic autonomous activity was noticed, while the presence of active begonia in particular stimulated an increased parasympathetic activity. Further, an increased spectral band was noted at 0.1 Hz, which indicates an increased systemic autonomous stability.

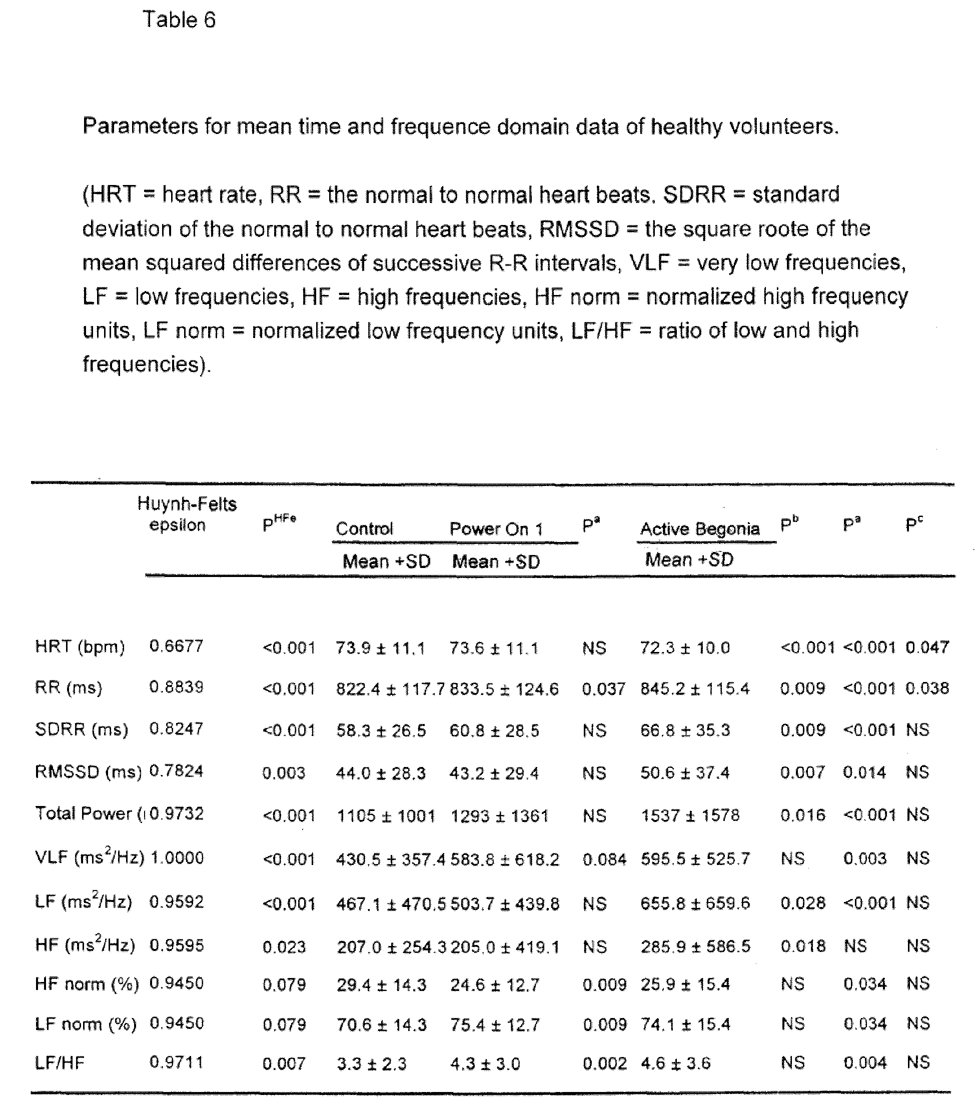

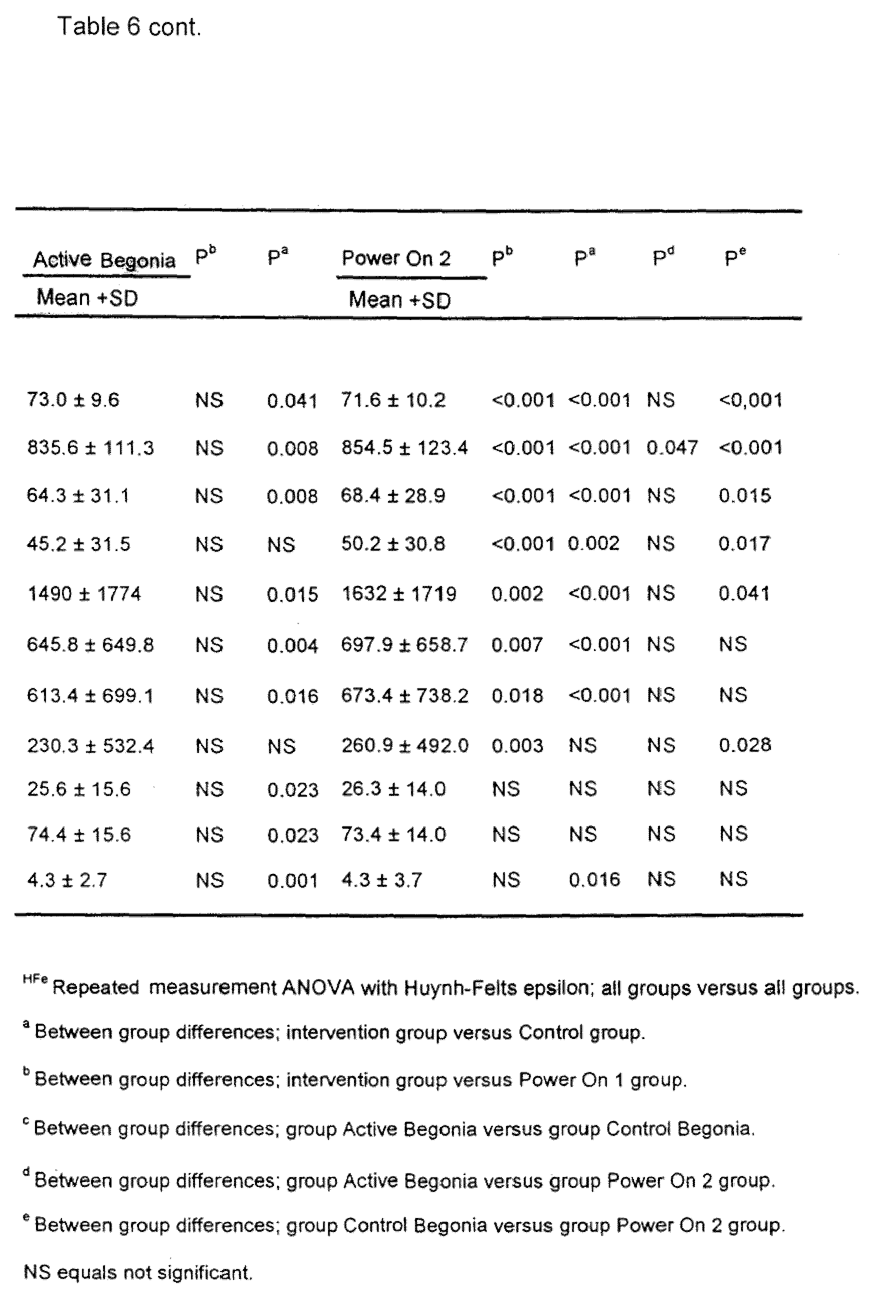

In correspondence with instantaneously adaptive life functions, such as heart rate, blood pressure, body temperature and respiration, also the immunological defence is reflexively autonomously regulated via vagus mediated mechanisms. Stimulation of the vagus nerve activates parasympathetic release of anti-inflammatory and anti-oxidative free-radical inhibiting substances and counteracts the effects of oxidative stress. Oxidative stress is characterized by several diversifiable external and host related stimuli, which together activate adaptive antioxidative defence lines for cellular protection against reactive oxygen compounds and the maintenance of an interstitial redox status. The cell's redox balance and protection against oxygen stress is maintained by co-operating enzymatic and non-enzymatic defence systems, which have the task of regulating the outcome of oxidative processes.

The direct and, respectively, indirect observed advantageous effect of synchronized water on yeast cells and oxidative metabolism of human beings, respectively, could thus be motivated from several hypothetical mechanisms or combinations of these; a) direct transfer of delocalized electrons or electron energy (free energy) from synchronized water to the electron transport chain in the mitochondrie, b) increased antioxidative capacity for the neutralisation of free oxygen radicals, in particular localized at the membrane surface, and ATP syntase, c) stabilization of the anti-clockwise rotation of ATP syntase, d) synergism and co-operativity reduces the surface tension, increases the solubility of substances, stabilizes enzymatic configuration and stimulates intra- and extracellular transport and enzymatic activity.

Thus, the studies indicate that the biosynthesis of mitochondrial energy under certain circumstances could be stimulated during exposure to synchronized water and complement, or alternatively replace, the production of hydride hydrogen in the glycolysis and citric acid cycle.

The following results were obtained, inter alia from the experiments performed above in the examples;
- evidence for macroscopical emergency at room temperature in synchronized water (self-regulating molecular synchronicity); 1) time-dependent conductivity increase with TGM and colloidal quartz, 2) time-dependent temperature reduction, and/or increased temperature stability (the mean value of the standard deviation was significantly lower in relation to control),
- synchronized water according to the present invention slows down the oxidation of foods and beverages, stabilizes proteins in solution (e.g. milk), stimulates enzymatic activity (trypsin catalysis) and counteracts denaturation of biological activity during exposure to UV, mobile and EMF (computer radiation),
- synchronized water stimulates the metabolism and production of stable anti-oxidative substances of yeast and adapts the redox activity to nutrient status,
- stabilized geometry and co-operative synergism leads to an increased organisation in synchronized water and makes the cellular oxidative energy yield more effective,
- adaptive autonomy with cardiovascular symphatovagal balance and increased vagus tone, which leads to increased autonomous stability, reduced physiological stress and stimulated secretory immunity.

### Example 14

Two men and two women were studied in a pilot experiment for tinnitus treatment with a matrix according to Figure 16F having an outer diameter of 34 mm and a line width of 0.1 mm. Said matrix was placed with a skin friendly adhesive locally at the cranial base behind the ear exhibiting the tinnitus symptoms. All of them had been suffering from the injury for several years and experienced substantial problems before the treatment.
1. Woman, 55-60 years, acupuncturist. The problems disappeared within 1 week.
2. Women, 55-60 years, dentist. The problems disappeared within 24 hours, very substantial improvement.
3. Man, 55-60 years, Managing Director. Substantial stress. The problems relating to both signal and pitch disappeared within 24 hours. Travelled abroad (by airplane). Was exposed to a high sound level on a night club and at a Formula 1 race. The tinnitus then returned. Treated again, and once more the problem disappeared within 24 hours.
4. Man, 45 years. Had suffered from tinnitus for 15 years. The problems relating to signal sound disappeared within 24 hours and periodically also the problems relating to the buzz.

Thus, the syncronized water according to the present invention may be used as a so-called functional water or a functional beverage for medical and health stimulating applications, such as for the prevention of ill-health via more efficient regulative homeodynamics, e.g. for stimulation of parasympatic activity and humoral immunity in connection with e.g. infections, for exhaustion problems and stress related problems, hypotonia and hypertonia, respectively, and for optimation of oxidative metabolism and energy utilization. A functional water or a functional food may be regarded as an effective food which during normal consumption consists of natural foodstuffs with documented physiological effect (34). This is related to diet factors influencing the human health condition with properties which may be measured objectively in the form of better opportunities for improved health and of an improved condition according to the subjective experience of the individual. The definition of health could be based on the fact that water constitutes a fundamental medium for optimal biological functional activity and exchange of information in vivo in the human and in relation to the living conditions in view of biochemical, electrophysiological, electromagnetic, cognitive and emotional information. The presence of water as a liquid or a gas results in interactivity with the autonomous nervous system of the human having effects on homeostasis, energy utilization and systemic recovery, in accordance with psycho-evolutionary theories (35), wherein natural stimuli induces and conditions self-regulative physiological and emotional autonomy of the human. Just like natural stimuli, synchronized water is permissive and attracts unconscious attention, a condition associated with physiologically recovery and behavioural flexibility with increased adaptivity against stress and the surrounding demands.

In one aspect the present invention relates to the use of a synchronized water as a functional water.

In another aspect the present invention relates to the use of a synchronized water for the preparation of a functional water for the prevention and/or treatment of hypertonia, hypotonia, diabetes of type I and II, exhaustion syndrome, inflammatory conditions and infections, such as Herpes simplex, wherein the synchronized water or the medium containing synchronized water optionally is administered together with a pharmaceutically acceptable medium.

In a further aspect the synchronized functional water may be used for peroval nutrition with a view to restoring functional homeostasis.

In a further aspect the synchronized water may be used as a preservation promoting additive in food products, e.g. dairy products.

Further, the technology could in another aspect be utilized with a view to conditioning the air via additional synchronized water in gas phase in opened or closed indoor environments, e.g. home environments or working areas, in particular working areas having a load of electromagnetic fields from computers and mobile telephones or in aeroplane cabins and other transport vehicles.

In further aspects the synchronized water could be used as a solution for the stabilisation of proteins, preferably enzymes, most preferably trypsine, as a storage solution for contact lenses and other eye liquids; as a means for the production of a stable anti-oxidative cocktail for use as a functional beverage or as an additive in ordinary beverages.

Thus, the synchronized water according to the present invention may be present in liquid-based pharmaceutical preparations and in hygiene products, e.g. in antibiotic preparations, insulin preparations, nasal sprays, foods, contact lens liquids, lotions, in particular in UV-light protective preparations, wherein the synchronized water may be administered to a mammal, in particular human beings, in any conventional pharmaceutical or non-pharmaceutical preparation form, such as a solution, a suspension, a paste, an ointment, a tablet, a spray, etc.

Further, the synchronized water may also be used as an additive to micro-organisms during the de-composition of bio-organic material, e.g. during the de-composition of undesired excess biomass material after the harvest of different crops, such as root vegetables, olives and organic waste. A problem in connection with present decomposition processes is that the decomposition proceeds slowly and that an unpleasant smell is spread to the surroundings. During application of the synchronized water according to the present invention together with the decomposing micro-organisms, the de-composition process is accelerated, and the undesired smell is reduced or eliminated. The positive effects obtained are believed to depend on increased growth of micro-organisms, as well as these micro-organisms having obtained an increased intended functional activity, wherein oxidative mouldering processes are made more effective and are accelerated.

Satisfactory experiments have been performed on waste biomass with a mixture of the synchronized water according to the present invention and a product called Terra Biosa. This product is produced by Biosa Danmark APS, Sonerupsvej 41, DK-3300 Frederiksverk and has the license No. 208-R884080 and AUT. No. 1081154. This product is a mixture of aromatic organic herbs which have been fermented by using a specific combination of lactic acid cultures. During the fermentation lactic acid is formed, which leads to a low pH of about 3.5. This low pH prevents the development of harmful bacteria in the end product.

The product contains, inter alia, organic molasses from sugar cane, organic fructose, organic dextrose, organic herbs and fermentation cultures, such as Lactobacillus acidophillus, Bifidobacterium animalis subsp. lactis, Streptococcus thermophilus, Lactobacillus casei, Lactococcus lactis subsp. lactis, Lactococcus lactis subsp. lactis biov. diacetyllactis and Leuconostoc pseudomesenteroides.

The present invention also relates to treatment of several of the disease conditions mentioned above by the provision of a normalisation of functional adaptivity of a human being or an animal by administering a preparation containing the synchronized water to the human being or the animal in need thereof.

The present invention also relates to a method for the treatment of tinnitus, wherein a topographic geometrical matrix, preferably a matrix according to Figure 16F, is placed on the body of a patient, preferably in the vicinity of the affected ear, wherein a synchronization of the water in the patient's body takes place.

The present invention has been described above with reference to preferred embodiments of the invention. However, a skilled person in the art realises that further variants within the scope defined by the present claims are intended to be included in the present invention.

### Literature

1. Cho CH, Singh S and Robinson GW. Liquid water and biological systems: the problem in science that hardly anyone wants to be solved. Faraday Discuss 103, 19-27, 1996.
2. Chaplin MF. A proposal for the structuring of water. Biophys Chem 83, 211-221, 2000.
3. Franks F. Introduction- water, the unique chemical. Ed. Franks F. Water a comprehensive treatise, Vol1 Plenum Press, New York, 1972, 1-20.
4. Lo SY. Survey of IE™ Clusters. In: Physical, chemical and biological properties of stable water (IE™) clusters. Proceedings of the first international symposium. Eds. SY. Lo and B. Bonavida. World Scientific Publishing Co. Pte. Ltd London, pp. 3-45,1998.
5. Auerbach D. Supercooling and the Mpemba effect; when hot water freezes quicker than cold water. Am J Phys 63, 882-885, 1995.
6. Luck WAP. Water and ions in biological systems. Eds. Pullman A, Vasileui V and Packer L. Plenum Press, New York, 1985, 95-122.
7. Robinson GW, Zhu SB, Singh S and Evans MW. Water in biology, chemistry and physics, Experimental overviews and Computational Methodologies, World scientific, Singapore, 1996.
8. Speedy RJ. Waterlike anomalies from repulsive interactions. J Chem Physics. 107, 1997, 3222-3229.
9. Liu K, Cruzan D and Saykally RJ. Water clusters. Science 271, 929-933, 1996.
10. Tsai CJ and Jordan KD. Theoretical study of small water clusters. J. Phys. Chem. 97, 1993, 5208-5210.
11. Watterson JG. The pressure pixel: unit of life. BioSystems 41, 1997, 141-152.
12. Lo SY, Li WC, Huang SH. Water clusters in life. Med Hypoth 2000;54;948-953.
13. Strogartr S. Sync How order emerges from chaos in the universe, nature and daily life. Theia, USA, 2004.
14. Vedamuthu M, Singh S and Robinson GW. Properties of liquid water: origin of the density anomalies. J. Phys. Chem. 98, 1994, 2222-2230.
15. Cho CH, Singh S and Robinson GW. Understanding of all water's anomalies with a non-local potential. J. Chem. Phys. 107, 1997, 7979-7988.
16. Robinson GW and Cho CH. Role of hydration water in protein unfolding. Biophys. J. 77, 1999, 3311-3318.
17. Graziano G. On the size dependence of hydrophobic hydration. J. Chem. Soc. 94, 1998, 3345-3352.
18. Steel EA, Merz KM, Selinger A and Castleman AW. Mass-spectral and computational free-energy studies of alkali-metal ion-containing water clusters. J. Phys. Chem. 99, 1995, 7829-7836.
19. Watterson JG. The interactions of water and protein in cellular function. Prog. Mol. Subcell. Biol. 12, 1991, 113-134.
20. Rand RP and Parsegian VA. Hydration force between phospholipid bilayers. Biochim. Biophys. Acta. 988, 1989, 351-376.
21. Wiggins PM, Enzymes and two state of water. J. Biol Phys. Chem. 2, 2002, 25-37.
22. Woutersen, S., & Baker, H. J. Resonant intermolecular transfer of vibrational energy in liquid water. Nature, 402, 1999, 507-509.
23. W. E. Dibble 8 W. A. Tiller, Development of pH and temperature and oscillations in water containg ZnCO3 crystallites using intention imprinted electronic devices, Subtle Energies & Energy Medicine 8,3, 1997, 175-193.
24. Coates C. Living Energies. Gateway Books, 1998.
25. Hanstorp D. Jakten på absoluta nollpunkten. Chalmers Magasin 3;2001;38-39.
26. Giddy AP, Dove MT, Pawley GS and Heine V. The determination of rigid unit modes as potential soft modes for displace phase transitions in framework crystal structures. Acta Crytallograph A49, 697-703, 1993.
27. Hammonds KD, Dove MT, Giddy AP and Heine V. Crush: a fortran program for the analysis of rigid unit mode spectrum of framework structure. Am Mineralog 79, 1207-1209, 1994.
28. Klein RA. Ab Initio Calculations of 17O NMR-chemical shifts for water. The limits of PCM theory and the role of hydrogen-bond geometry and cooperativity. J Phys Chem A 2004, 108, 5851-5863.
29. a) Tiller W, Dibble WE, Kohane MJ. Conscious acts of creation. Pavior Publishing, Walnut Creek, California, US, 2001. 29b) Korotkov K and Korotkin A.. Concentration dependence of gas discharge around drops of inorganic electrolytes. J Appl Physics 89, 4732-4739, 2001.
30. Agladze KI and Krinsky VI. Multi-armed vortices in an active chemical medium. Nature 296, 424-426, 1982.
31. Walleczek J. Nonlinear dynamics, selforganisation and biomedicine. Cambridge university press, UK, 1999.
32. Bassingthwaighte JB et al. Fractal Physiology. Oxford university press, NY, 1994.
33. Pinto V. The relative hydrogen score, aka the rH score, www.h-minus-ion.orolrH-score-1.html
34. Diplock AT, Aggret PJ. Scientific concepts of functional foods in Europe. Consensus document. Br J Nutr 81, Suppl 1:S1-S27, 1999.
35. Easterbrook Ja. The effect of emotion on cue utilization and the organisation of behavior. Psychol Review 66, 183-201, 1959.
36. Liu K, CruZan D, and Saykally RJ. Water clusteras. Science 271, 929-933, 1996.
37. Tsai CJ, and Jordan KD. Theoretical study of small water clusters. J Phys Chem 97, 5208-5210, 1993.
38. Lo SY, Li WC and huang SH. Water clusters in life. Med Hypothesis 54, 948-53, 2000.
39. Teschke o and de Souza EF. Water molecule clusters measured at water/air interfaces using atomic force microscopy. Phys Chem Chem Phys 7, 3856-3865, 2005.
40. Bulienkov NA. The role of system-forming modular water structures in self-organization of biological systems. J Molec Liquids 106, 257-275, 2003.
41. Higashitani, K., Kage, A., Katamura, S., Imai, K., Hatade, S. Effects of a magnetic field on the formation of CaCO3 particles. Journal of colloid and interface science 156 (1993) 90-95.
42. Kney, A.D., Parsons, S.A. 2006. A spectrophotometer-based study of magnetic water treatment: Assessment of ionic vs. surface mechanisms. Water research 40 (2006) 517-524.
43. Kronholm J. 2008 The permattivity in synchronized water. Internal Report 2008.
44. Garcia RA, et al. Detection of periodic signatures in the solar power spectrum. On the track of I=1 gravity modes. Astro Phys 27 Nov 2006.
45. Tiller WA. Dibble EW, Fandel JG. Some science adventures with real magic. Pavior Publishing California, US, 2005.
46. Tiller WA, Dibble WE, Nunley R, Shealey CN. Toward experimentation and discovery in conditional laboratoty spaces: Part I. Experimental pH change findings at some remote sites; J Altern Compl Med 10, 145-157, 2004.
47. Tiller WA, Dibble WE, Nunley R, Shealey CN. Toward experimentation and discovery in conditional laboratoty spaces: Part II. pH change experience at four remote sites; J Altern Compl Med 10, 301-306, 2004.

### EMBODIMENTS

1. A synchronized water, in which all single water molecules at the same time are arranged in an identical way to a stable homogeneous microstructure, wherein said synchronized water in a distilled condition and at atmospheric pressure has
   a) a density of from 0.997855 to 0.998836 g/ml at 22°C,
   b) a water temperature of from -6.7°C to -8.2°C at the freezing point,
   c) a melting point of from 0.1°C to 0.2°C,
   d) a surface tension of from 72.3 to 72.7 dyn/cm at 22°C, and
   e) a dielectric constant of from 82.4 to 82.6 F/m.
2. The synchronized water according to embodiment 1, wherein it also has a magnetic oscillatory frequence range of 4-50 Hz in vacuum.
3. The synchronized water according to embodiment 1, wherein it during exposure to daylight at room temperature during 10 h shows an average temperature increase of at most 0.1°C.
4. The synchronized water according to embodiment 1, wherein it in a suspension of spherical particles of colloidal quartz having a diameter of 6-8 nm each in the concentration range of 25-50 µg/ml shows a conductivity increase of ≥ 7 µS/cm.
5. The synchronized water according to any one of the proceeding embodiments, wherein it in relation to its original non-synchronized condition under otherwise identical conditions at the same time shows a changed pH, a reduced redox potential, a reduced relative hydrogen, and a reduced dissipative geometrical entropy.
6. The synchronized water according to any one of the proceeding embodiments, wherein quartz is present in the synchronized water with a view to promoting the stability thereof, preferably in colloidal form or in crystal or particle form.
7. A medium containing the synchronized water according to any one of embodiments 1-6.
8. The medium according to embodiment 7, wherein the concentration of the synchronized water in the medium is 60-100 vol%, preferably 80-100 vol%, based on the total amount of the medium.
9. The medium according to any one of embodiments 7 and 8, wherein it comprises or constitutes a food product, a functional medium, a preparation form for a medicament, a preservative, a cell-culturing solution, an enzyme substrate, or a gaseous space containing steam, preferably ordinary air.
10. The medium according to embodiment 9, wherein it is a dairy product, a soft drink (lemonade), a functional beverage, a bakery product, fruit and vegetables, functional foods, a galenic dosage form, preferably a solution, a suspension, an ointment, a syrup or a paste; a liquid intended for cleaning of contact lenses; a liquid intended for preservation of foods; mineral water, tap water, salt water and fresh water having varying salt concentrations in their natural forms, water intended for different industrial purposes and water intended for irrigation of plants and plant culturing.
11. A method for the preparation of synchronized water having the properties disclosed in embodiment 1, wherein light having a wavelength of 360-4000 nm is made to pass through a topographic geometrical matrix and thereafter is brought in contact with water or a water-containing medium, wherein the topographic geometrical matrix has the ability to influence the incident light in such a way that the water is synchronized and thereby obtains the properties defined in embodiments 1-5.
12. The method according to embodiment 11, wherein the topographic geometrical matrix has a design which is based on the geometry of the circle and/or the sphere.
13. The method according to embodiment 12, wherein the topographic geometrical matrix is a circle, a circle enclosing one or more concentric circles having a common center or a common tangential point on the arc of the circle, wherein the position for each concentrical circle follows the vertical axis Y1-Y2 in Figure 16A, f, l/f or fn, a circle containing a smaller closed circle, a circle containing several concentric circles, wherein one or more of the rings formed therein are closed; wherein in the case of concentrical circles the relationship θ (phi) applies between the outer diameter of the circle and the next circle counting inwards toward the common center of the circles, i.e. 1.68 or 1/θ, or in accordance with Fibonacchi's sequence of numbers, wherein Fn = θⁿ/5^{0.5} and powers often thereof, wherein the topographic geometrical matrix preferably is an SS matrix, as well as deviations, combinations and variants thereof, which during the radiation leads to synchronized water having the properties defined in embodiments 1-5.
14. The method according to embodiment 11, wherein the topographic geometrical matrix has the form of a quadrated circle, an equilateral triangle, a hexagram, a standing or lying giving sign, a squared circle, a pentagram; an equilateral triangle, a quadrated circle, a pentagram, a pentagon, a hexagon, an equilateral rhomb, a logarithmic spiral based on the geometry of θ; a matrix formed according to a Fibonacchi θ spiral; a triangulated hexagon, a triangulated hexagram; a star tetrahedron; and a Metatronic cube (platonic bodies); a normal sphere, a sphere containing a smaller open or closed sphere; a sphere containing one or more concentric spheres having a common center or a common tangential point on the surface of the sphere; a cube; an okta-hedron; a rhombogramme; a cylinder, optionally having uncoupled circles or an uncoupled torus spiral arranged on the surface of the cylinder, several similar or non-similar cylinders connected in groups; a cylinder having a concave or convex form or an eggform,
   as well as deviations, combinations and variants thereof, which during the radiation leads to synchronized water having the properties defined in embodiments 1-5.
15. The method according to embodiments 11-14, wherein the topographic geometrical matrix fully or partially is colored with one or more metallic colors.
16. The method according to embodiments 11-14, wherein the topographic geometrical matrix is designed in such a way that it has a maximum width in the range of nanometers up to micrometers, preferably 0.1 nm - 900 µm.
17. The method according to embodiments 11-14, wherein the topographic geometrical matrix is designed in such a way that one or more of the lines included in or constituting the matrix has a width of 2 nm - 2.0 mm, preferably 0.01-1.0 mm.
18. The method according to embodiment 11, wherein the topographic geometrical matrix is freely present in front of the surface of the water-containing medium to be radiated.
19. The method according to embodiment 11, wherein the topographic geometrical matrix is arranged on a support, which is made of a material which does not modify the electromagnetic properties of the incident light, preferably glass, cardboard, paper, plastic, sheet metal or natural material.
20. The method according to embodiment 11, wherein the topographic geometrical matrix is plated, imprinted, preferably with a letterpress or gold-leaf or silver-leaf; etched, glued or laminated on the support.
21. The method according to embodiment 19, wherein the glass is an optical cover glass of boron silicate or optical quartz glass.
22. The method according to embodiment 19, wherein the support constitutes a delimiting side of a container in which the water-containing medium to be radiated is present.
23. The method according to embodiment 19, wherein the support constitutes a delimiting surface of a flask, a bottle, a tank, a food package or a test tube.
24. The method according to embodiment 11, wherein the water-containing medium to be radiated is stationary or in motion, preferably during vortextreatment or in a flowing state.
25. The method according to embodiment 11, wherein a water-containing medium in motion in a process line is radiated.
26. The method according to embodiment 11, wherein air containing steam in a space is radiated.
27. The method according to embodiment 11, wherein the radiation is performed by use of a spectrophotometer, daylight, a full-light lamp, a diod or a spectral filter.
28. The method according to embodiment 11, wherein quartz is present in the water-containing medium during the radiation with a view to increasing the stability of the formed synchronized water, preferably in colloidal form or in crystal or particle form.
29. The method according to embodiment 11, wherein the topographic geometrical matrix is made of a metal, preferably copper or brass.
30. Use of the synchronized water according to any one of embodiments 1-6 or the medium according to any one of embodiments 7-10 for the preparation of a functional medium for the prevention and/or treatment of hypertonia, hypotonia, type 1 and 2 diabetes, exhaustion syndrome, inflammatory conditions and infections, preferably of Herpes simplex, optionally together with a pharmaceutically acceptable medium.
31. Use of the synchronized water according to any one of the embodiments 1-6 or the medium according to any one of embodiments 7-10 as a preservation promoting additive in foods, preferably dairy products.
32. Use of the synchronized water according to any one of embodiments 1-6 or the medium according to any one of embodiments 7-10 as a solution for stabilization of proteins, preferably enzymes, most preferably trypsine.
33. Use according to anyone of embodiments 1-6 or the medium according to any one of embodiments 7-10 as a storage solution for contact lenses and other eye liquids.
34. Use according to any one of embodiments 1-6 or the medium according to any one of embodiments 7-10 for the improvement of the reductive and anti-oxidative capacity of micro-organisms, preferably yeast cells, or for the production of a stable and anti-oxidative cocktail for use as a functional beverage for peroral nutrition or as an additive in ordinary beverages.
35. Use of the synchronized water according to embodiments 1-6 or the water-containing medium according to embodiments 7-10 as an additive to micro-organisms during the composition of bio-organic material with a view to accelerating the mouldering process and reducing the smell intensity.
36. Use of the synchronized water according to any one of embodiments 1-6 or the medium according to any one of embodiments 7-10 for the improvement and conditioning of the air quality in indoor environments, preferably in cabin environments, most preferably in aeroplanes.
37. Use of the synchronized water according to any one of embodiments 1-6 or the medium according to any one of embodiments 7-10 for the protection against UV, mobile telephone and EMF radiation.
38. A method for the treatment of the disease conditions defined in embodiment 30 by the provision of a functional normalized adaptivity in a human or an animal, wherein a preparation containing the synchronized water according to any one of embodiments 1-6 or the water-containing medium according to any one of embodiments 7-10 is administered to the human being or the animal in need thereof.
39. A device comprising a topographic geometrical matrix and a support as defined in any one of embodiments 19-23, wherein the topographic geometrical matrix is an SS matrix having the form of an open circle concentrically enclosing a closed smaller inner circle, wherein the diameter of the outer circle is 2 nm - 987 µm, the diameter of the inner circle is 0.125 nm - 233 µm, and the line width of the outer circle is 0.125 nm - 8 µm, wherein said diameters and band width are mutually related according to Fibonacchi's sequence of number, and wherein the topographic geometrical matrix is applied on the support, which preferably is transparent.
40. The device according to embodiment 39, wherein the support is a plaster.
41. A method for therapeutic treatment of tinnitus, wherein a topographic geometrical matrix as defined in any one of embodiments 11-17 and 39-40 is applied on the skin of a patient's body, preferably in the vicinity of the affected ear, and is subjected to radiation with daylight.

## Claims

1. A tinnitus treatment device comprising a topographic geometrical matrix and a support wherein the topographic geometrical matrix is applied on the support and has the form of an open circle concentrically enclosing a smaller inner circle (an SSc matrix), wherein the relationship between the circle diameters follows Fibonaochi's sequence of numbers, or has the form of the flower of life.

2. The device according to claim 1, wherein the support is made of a material that does not modify the electromagnetic properties of incident light, wherein said material preferably is glass, cardboard, paper, plastic, sheet material or natural material.

3. The device according to claim 1, wherein the topographic geometrical matrix is plated, imprinted, etched, glued or laminated on the support.

4. The device according to claim 2, wherein the support is a plaster.

5. The device according to claim 2, wherein the support is transparent.

6. The device according to any one of the preceding claims, wherein it has the ability to be applied on the skin of a patient's body, preferably in the vicinity of a tinnitus affected ear.
